(19) Europäisches Patentamt
European Patent Office
Office européen des brevets

(11) Publication number: **0 230 035 B1**

# (12) EUROPEAN PATENT SPECIFICATION

(45) Date of publication of patent specification: **12.10.94**

(51) Int. Cl.$^5$: **C07D 403/12**, C07D 401/12, A61K 31/40, A61K 31/415, A61K 31/435

(21) Application number: **86117878.8**

(22) Date of filing: **22.12.86**

The file contains technical information submitted after the application was filed and not included in this specification

## (54) Omega-[(hetero)alkyl]benz[cd]indol-2-amines.

(30) Priority: **13.01.86 US 818315**

(43) Date of publication of application:
**29.07.87 Bulletin 87/31**

(45) Publication of the grant of the patent:
**12.10.94 Bulletin 94/41**

(84) Designated Contracting States:
**AT BE CH DE ES FR GB GR IT LI NL SE**

(56) References cited:
**US-A- 4 259 490**
**US-A- 4 261 896**

**COLLECTION CZECHOSLOVAK CHEMICAL COMMUNICATIONS, vol. 50, pages 1888-1898, 1985; M. PROTIVA et al.:"Cyclic amidines derived from benz (c,d) indole and 4,5-dihydro-3H-1-benzazepine including some related compounds: synthesis and pharmacological screening"**

(73) Proprietor: **AMERICAN CYANAMID COMPANY**
**One Cyanamid Plaza**
**Wayne, NJ 07470-8426 (US)**

(72) Inventor: **Tomcufcik, Andrew Stephen**
**48 Dearborn Drive**
**Bergen, N.J. 07675 (US)**
Inventor: **Meyer, Walter Edward**
**40 Van Orden Avenue**
**Suffern, N.Y. 10901 (US)**
Inventor: **Chan, Peter Sinchun**
**166 Wayne Avenue**
**Suffern, N.Y. 10901 (US)**
Inventor: **Crandall, David Leroy**
**1B Timber Drive**
**Highland Mills, N.Y. 10930 (US)**

(74) Representative: **Wächtershäuser, Günter, Prof. Dr.**
**Patentanwalt**
**Tal 29**
**D-80331 München (DE)**

**EP 0 230 035 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

US-A-4,261,896 discloses 2-(substiuted-imino)-dihydro benz(cd)indoles and US-A-4,259,490 discloses 2-(substituted amino)benz(cd)indoles which differ structurally from those of the present invention. Coil. Czechoslovak Chem. Comm. 50(1985),p. 1888-1998 discloses 2-(subst.Amino)benz(cd)indoles which are unsubstituted on the benzindole moiety.

This invention is concerned with novel substituted and unsubstituted omega-[(hetero)alkyl]-benz[cd]-indol-2-amines which may be represented by the structural formula:

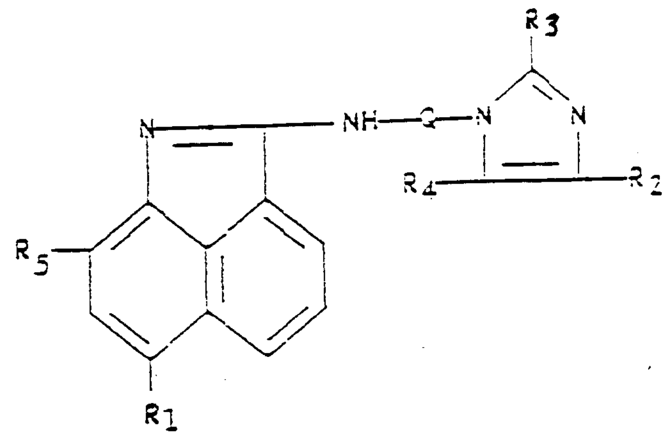

wherein $R_1$ is hydrogen, bromo, chloro or dimethylsulfonamide; $R_2$ is hydrogen or alkyl($C_1$-$C_3$); $R_3$ is hydrogen, alkyl($C_1$-$C_3$) or phenyl; $R_4$ is hydrogen or when taken together with $R_2$ is -CH=CH-CH=CH-; $R_5$ is hydrogen or chloro; and Q is $-(CH_2)_n-$, where n is an integer from 2 through 12,

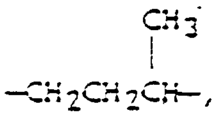
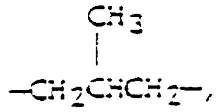
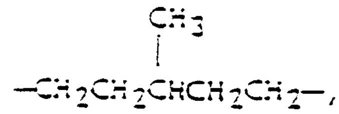
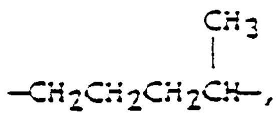
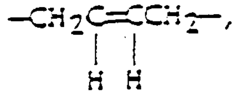
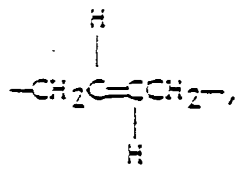
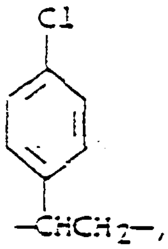
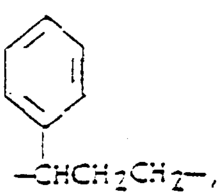
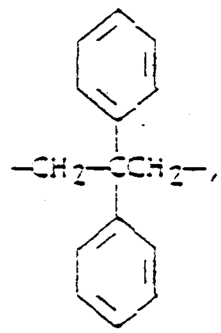

$-CH_2CH_2-O-CH_2CH_2-$, 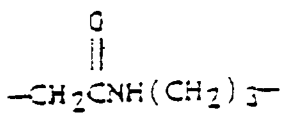 or 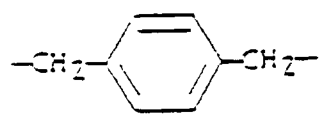

and the pharmacologically acceptable salts thereof.

Further, the present invention relates to the novel compounds defined in Claims 2 to 7.

The organic bases of this invention form non-toxic acid-addition salts with a variety of pharmacologically acceptable organic and inorganic salt-forming reagents. Thus acid-addition salts, formed by admixture of the organic free base with one or more equivalents of an acid, suitably in a neutral solvent, are formed with such acids as sulfuric, phosphoric, hydrochloric, hydrobromic, hydriodic, sulfamic, citric, lactic, malic, succinic, maleic, fumaric, tartaric, acetic, benzoic, gluconic, ascorbic and the like. For the purpose of this invention the free bases are equivalent to their non-toxic acid-addition salts.

## DETAILED DESCRIPTION OF THE INVENTION

The compounds of the present invention may be readily prepared according to the following reaction schemes, wherein $R_1$, $R_2$, $R_3$, Q, are as described hereinabove.

Scheme 1:

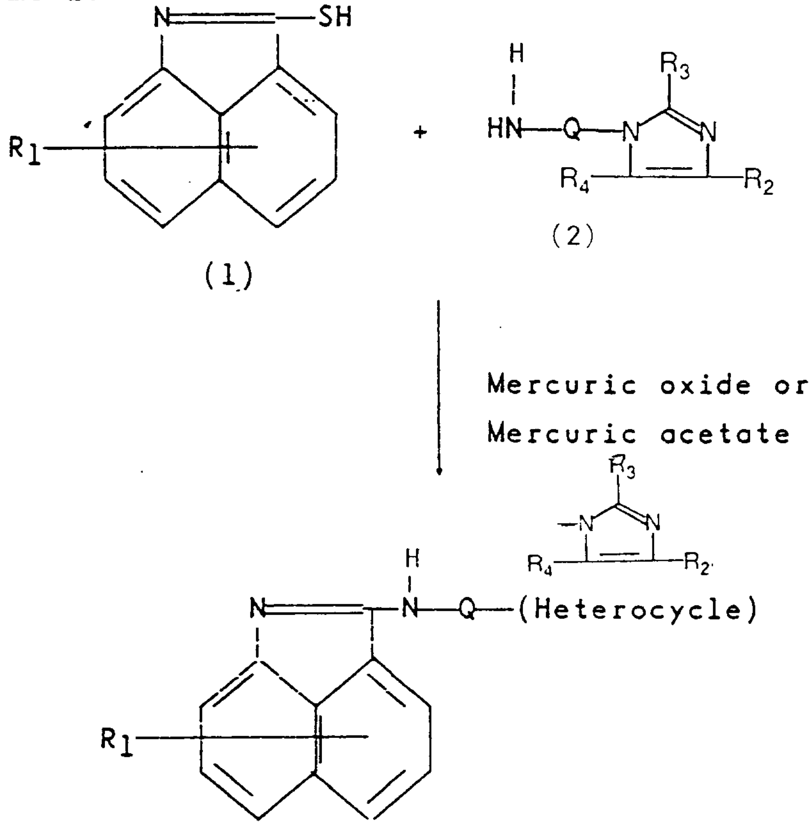


(1) (2) (3)

In accordance with the above reaction scheme a substituted bens[cd]indol-2-thiol (1) is reacted with an amine of the general structure (2) and mercuric oxide or mercuric acetate in a suitable solvent such as ethanol, butanol, or 2-methoxyethanol at reflux temperature for several hours, giving the desired compounds.

The compounds of Structure (1) are readily prepared by the following reaction:

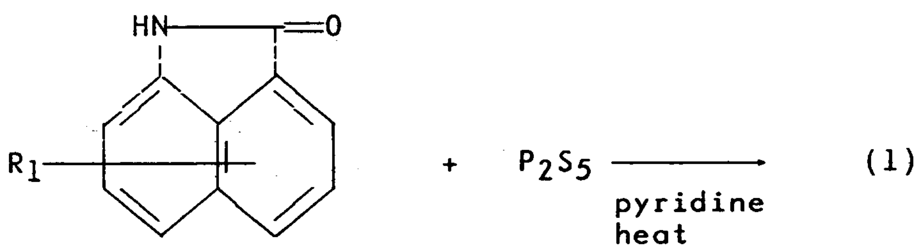


(1)

(4)

Compounds of Structure (4) are well known in the literature, and are prepared as shown in Great Britain Patent 1,595,050, United States Patent 2,628,964, West Germany Patent DE 3,443,994, Helv. Chim. Acta 34 382 (1951), J. Org. Chem. USSR 7 150 (1971) and 8 826 (1972), and others. The conversion of compounds of Structure (4) to those of Structure (1) is readily accomplished as described in the J. Chem. Soc. 1960 1537 and J. Gen. Chem. USSR 24 1871 (1954).

The compounds of Structure (2) are also well known in the literature and are prepared by the methods and procedures as exemplified in United States Patents 4,551,460 and 4,568,687, Helv. Chim. Acta 65 1868 (1982), J. Het. Chem. 10 39 (1973), Eur. J. Med. Chem. - Chim. Ther. 1985-20 (S) p. 403, and J. Med. Chem. 29 2280 (1986), and others.

Scheme 2:

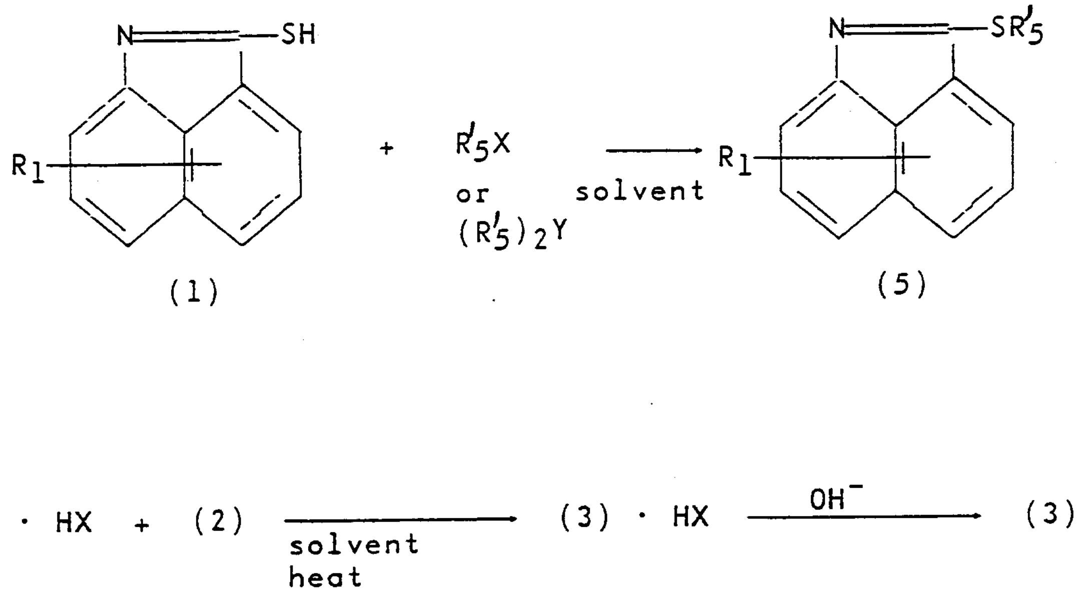


In accordance with the above scheme, a benz[cd]indol-2-thiol derivative of Structure (1) is dissolved in a solvent such as acetone, ethanol and the like and treated with a slight excess of an alkylating agent $R'_5X$ or $(R'_5)_2Y$ (where $R'_5$ is alkyl or arylaklyl; X is halo; and Y is sulfate), such as iodomethane, bromoethane, dimethyl sulfate, benzylchloride and the like, yielding a 2-substituted thiobenz[cd]indole salt (Structure 5), as discussed in J. Chem. Soc. 1960 1537. The latter compounds when treated with the amines of Structure (2) in an appropriate solvent such as ethanol or 2-methoxyethanol yield the HX salts of the compounds of Structure (3) which when neutralized with alkali hydroxides yield the free bases (Structure (3)).

Scheme 3:

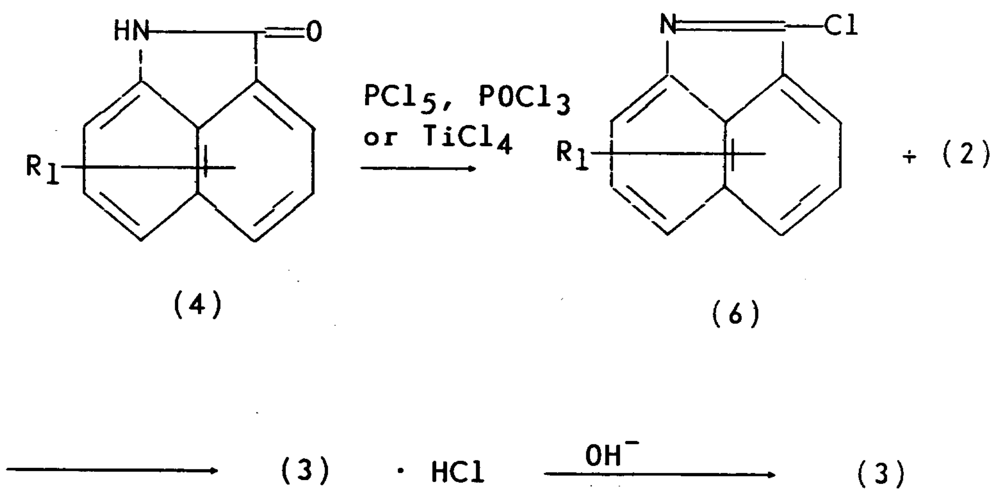


By this reaction scheme, compounds of Structure (4) when treated with $PCl_5$, $POCl_3$, $TiCl_4$, and the like in a suitable solvent can yield a 2-chloro-benz[cd]indole of Structure (6), which upon treatment with (2), followed by treatment with alkali hydroxides can yield the free base (Structure (3)).

Scheme 4:

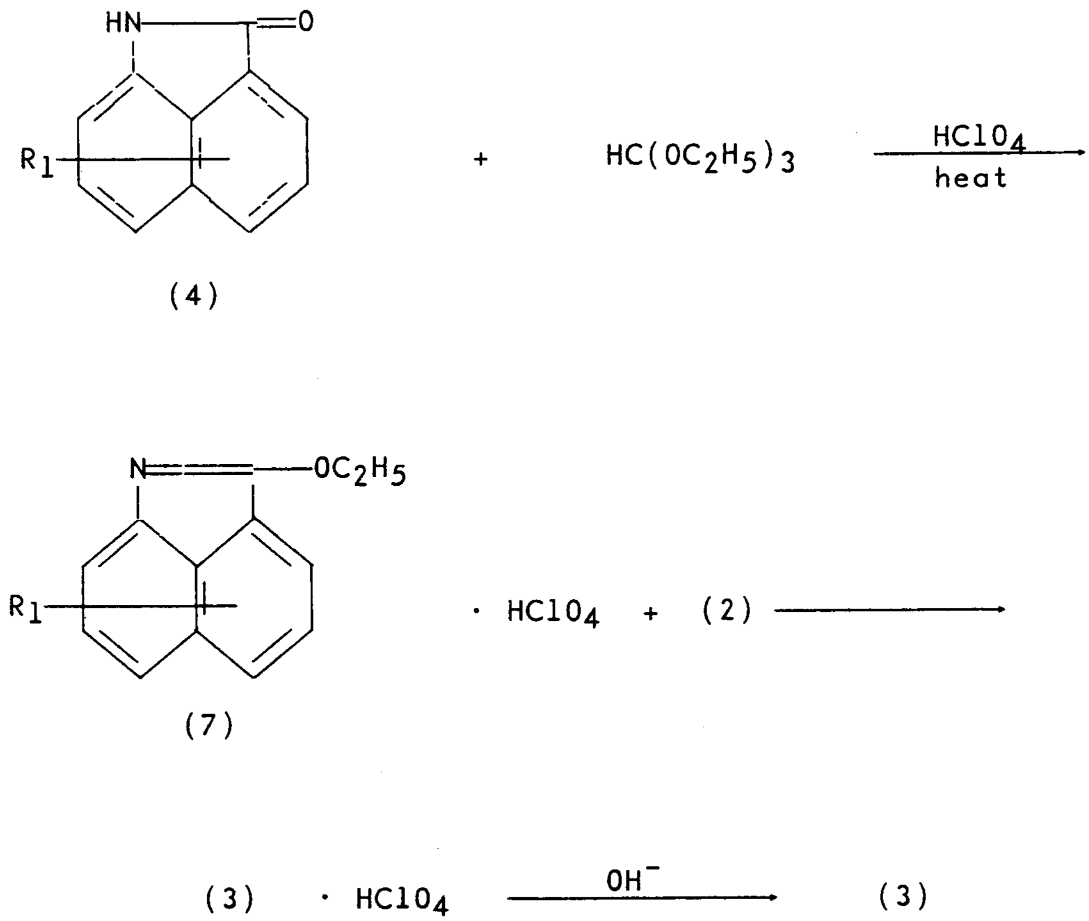


(4)

(7)

(3) $\cdot\ HClO_4$ → (3)

According to this reaction scheme, compounds of Structure (4) when treated with triethylorthoformate in the presence of perchloric acid can yield 2-ethoxy-benz[cd]indole perchlorate salts of Structure (7) which when treated with compounds of Structure (2) can yield the perchlorate salts of compounds of Structure (3), which on treatment with alkali hydroxide can give the compounds of Structure (3). The reaction of (4) with triethylorthoformate in the presence of perchloric acid is exemplified in J. Org. Chem. USSR 17 (10) 2225 (1981).

The compounds of this invention inhibit thromboxane synthetase enzyme. Thus, these compounds are useful in the treatment of diseases characterized by an imbalance of thromboxane $A_2$/prostacyclin such as ischemic heart disease, transient ischemic attack, thrombosis and migraine. Recent reviews have established the role of the thromboxane/prostacyclin balance in the vascular system [Cardiovascular Diseases: New Trends in Surgical and Medical Aspects, H. Barnett, P. Paoletti, E. Flamm and G. Brambilla, eds., Elsevier/North-Holland Biomedical Press, pp 137-150 (1981)]. Prostacyclin ($PGI_2$) is a potent vasodilator and platelet aggregation inhibitor, whereas thromboxane ($TXA_2$) is a powerful vasoconstrictor and causative of platelet aggregation. $TXA_2$ is synthesized by thromboxane synthetase enzyme located in, for example, blood platelets. When $TXA_2$ production is increased relative to $PGI_2$, platelet aggregation, thrombosis and vasospasm may occur [Lancet (i), 1216 (1977); Lancet, 479 (1977); Science, 1135 (1976); Amer. J. Cardiology, 41 787 (1978)]. $TXA_2$ synthetase inhibitors have been shown to have superior anti-thrombotic action to that of aspirin [J. Clin. Invest., 65 400 (1980); Br. J. Pharmac., 76, 3 (1982)].

The role of prostaglandins including $TXA_2$ and $PGI_2$ in ischemic heart patients has been reviewed [Cardiovascular Pharmacology of the Prostaglandins, A. G. Herman, P. M. Vanhoute, H. Denolin and A. Goosens, eds., Raven Press, New York, pp 361-374 (1982)]. Injection of $TXA_2$ into coronary arteries of guinea pigs and rabbits causes myocardial ischemia and subendocardial necrosis [Drugs of the Future, 7, 331 (1982); Proc. Jap. Acad., 53(B), 38 (1977); Eur. J. Pharmacol., 53 49(1978)]. Recent research has

demonstrated the beneficial effects of $PGI_2$ and selective inhibition of thromboxane synthetase on ischemic myocardium in canines [J. Cardiovascular Pharmacology, 4, 129 (1982)]. Thus compounds which selectively inhibit thromboxane synthetase (and hence $TXA_2$) without adversely affecting $PGI_2$ are useful in the treatment of vascular diseases such as ischemia and migraine. In addition, inhibition of $TXA_2$ formation may effectively treat platelet aggregation and prevent thrombosis.

Under urethan anesthesia, (1 g/kg i.p.), 9.0 ml of arterial blood was collected through a cannula inserted into the carotid artery in 1 ml of 3.2% sodium citrate into a polystyrene tube from a male Okamoto-Aoki spontaneously hypertensive rat (Taconic Farms, Germantown, NY) between 19 and 24 weeks of age. The blood was diluted with 3 ml cold saline and centrifuged at room temperature for 15 min at 468 x g. The platelet rich plasma (PRP) was separated. The platelets were isolated by centrifuging the PRP for 10 minutes at 1060 x g and washed in 4 ml cold oxygenated Krebs phosphate buffer, pH 7.4. The chilled platelets recovered from centrifuging at 800 x g for 10 minutes were resuspended in oxygenated Krebs phosphate buffer and diluted to contain 4.0-6.0 x $10^4$ platelets/micro-liters.

The inhibition of TX formation was studied by determining the concentration of thromboxane $B_2$ ($TXB_2$)-,the stable hydrolysis product of $TXA_2$. Assay samples prepared on ice, contained 200 micro-liters platelet suspension, 50 micro-liters saline, and 50 micro-liters vehicle (saline) or drug under study. The test drug (0.003 mole) was dissolved in 5 ml of saline. Serial dilutions of the test drug solution were made with 0.9% saline to give assay concentrations of 1 x $10^{-4}$ to 1 x $10^{-9}$ mole.

The assay samples were incubated for 10 minutes at 37°C in a metabolic shaker at about 60 rpm. The reaction was terminated by immersing the tubes in an ice bath and adding 50 micro-liters of 0.5 M citric acid. The samples were centrifuged for 10 minutes at 2000 rpm at 4°C, and the supernatants were decanted. The $TXB_2$ content in each sample was determined by a direct radioimmunoassay (RIA) utilizing a $TBX_2$ specific RIA kit purchased from New England Nuclear, Boston, MA, and expressed as pg $TXB_2$ formed minute$^{-1}$, sample$^{-1}$, from which the percent inhibition of $TXB_2$ formation was calculated.

Table I shows the percentage of thromboxane synthetase enzyme inhibition when the compound was employed at an assay concentration of 1 x $10^{-4}$ Molar.

TABLE I

Thromboxane Synthetase Enzyme Inhibition

| Compound | % Inhibition |
|---|---|
| N-[3-(1H-Imidazol-1-yl)propyl]benz[cd]-indol-2-amine, dihydrochloride | 85 |
| 6-Bromo-N-[3-(1H-imidazol-1-yl)propyl]-benz[cd]indol-2-amine, dihydrochloride | 85 |
| N-[3-(1H-Imidazol-1-yl)butyl]benz[cd]-indol-2-amine, dihydrochloride | 92 |
| N-[1-(4-Chlorophenyl)-2-(1H-imidazol-1-yl)ethyl]benz[cd]indol-2-amine, fumarate | 35 |
| N-[3-(1H-Imidazol-1-yl)-2-methylpropyl]-benz[cd]indol-2-amine, dihydrochloride | 95 |
| N-[3-(1H-Imidazol-1-yl)-1-phenylpropyl]-benz[cd]indol-2-amine, fumarate | 100 |
| N-[3-(1H-Imidazol-1-yl)-2-methylpropyl]-benz[cd]indol-2-amine, fumarate | 100 |
| N-[5-(1H-Imidazol-1-yl)pentyl]benz[cd]-indol-2-amine, fumarate | 95 |
| (Z)-N-[4-(1H-Imidazol-1-yl)-2-butenyl]-benz[cd]indol-2-amine, dihydrochloride | 97 |
| N-[3-(2-Phenyl-1H-imidazol-1-yl)propyl]-benz[cd]indol-2-amine, hydriodide | 100 |
| N-[3-(2-Methyl-1H-imidazol-1-yl)propyl]-benz[cd]indol-2-amine, hydriodide | 82 |
| N-[4-(1H-Imidazol-1-yl)butyl]benz[cd]-indol-2-amine, hydriodide | 100 |
| (Z)-N-[4-(1H-Imidazol-1-yl)-2-butenyl]-benz[cd]indol-2-amine, hydriodide | 100 |
| (E)-N-[4-(1H-Imidazol-1-yl)-2-butenyl]-benz[cd]indol-2-amine, hydriodide | 100 |
| N-[3-(1H-Benzimidazol-1-yl)propyl]benz-[cd]indol-2-amine, fumarate | 90 |

TABLE I (Continued)

| Compound | % Inhibition |
|---|---|
| N-[3-(1H-Benzimidazol-1-yl)propyl]benz-[cd]indol-2-amine | 60 |
| N-[4-(1H-Imidazol-1-yl)butyl]benz[cd]-indol-2-amine, fumarate | 100 |
| N-[5-(1H-Imidazol-1-yl)-3-methylpentyl]-benz[cd]indol-2-amine, dihydrochloride | 100 |
| N-[10-(1H-Imidazol-1-yl)decyl]benz[cd]-indol-2-amine, fumarate | 65 |
| N-[2-(1H-imidazol-1-yl)ethyl]benz[cd]-indol-2-amine | 71 |
| 6-Bromo-N-[3-(1H-imidazol-1-yl)butyl]-benz[cd]indol-2-amine | 100 |
| N,N-Dimethyl-2-{[3-(1H-imidazol-1-yl)-butyl]amino}benz[cd]indol-6-sulfonamide | 99 |
| N,N-Dimethyl-2-{[3-(1H-imidazol-1-yl)-propyl]amino}benz[cd]indol- 6-sulfonamide | 100 |
| 6-Bromo-N-[10-(1H-imidazol-1-yl)decyl]-benz[cd]indol-2-amine | 94 |
| 6-Bromo-N-[4-(1H-imidazol-1-yl)butyl]-benz[cd]indol-2-amine | 98 |
| N-{[4-(1H-Imidazol-1-ylmethyl)phenyl]-methyl}benz[cd]indol-2-amine, fumarate | 100 |
| 6,8-Dichloro-N-[10-(1H-imidazol-1-yl)-decyl]benz[cd]indol-2-amine | 96 |
| 6,8-Dichloro-N-[3-(1H-imidazol-1-yl)-propyl]benz[cd]indol-2-amine | 97 |
| 6-Bromo-N-[5-(1H-imidazol-1-yl)pentyl]-benz[cd]indol-2-amine | 97 |

TABLE I (Continued)

| Compound | % Inhibition |
|---|---|
| 6-Chloro-N-[5-(1H-imidazol-1-yl)-pentyl]benz[cd]indol-2-amine | 100 |
| N-[4-(1H-imidazol-1-yl)pentyl]benz-[cd]indol-2-amine, fumarate | 99 |
| 6-Chloro-N-[4-(1H-imidazol-1-yl)butyl]-benz[cd]indol-2-amine, fumarate | 100 |
| N-[3-(1H-Imidazol-1-yl)-2,2-diphenyl-propyl]benz[cd]indol-2-amine | 75 |
| N-{2-[2-(1H-Imidazol-1-yl)ethoxy]-ethyl}benz[cd]indol-2-amine bis-fumarate | 98 |
| N-[8-(1H-Imidazol-1-yl)octyl]benz[cd]-indol-2-amine dihydrochloride | 100 |
| N-[4-(1H-Imidazol-1-yl)pentyl]benz-[cd]indol-2-amine, bis-fumarate | 99 |
| 6,8-Dichloro-N-[4-(1H-imidazol-1-yl)-butyl]benz[cd]indol-2-amine | 99 |
| N-[3-(1H-Imidazol-1-yl)propyl]benz-[cd]indol-2-amine, fumarate | 92 |
| N-[12-(1H-Imidazol-1-yl)dodecyl]-benz[cd]indol-2-amine | 97 |
| 6-Bromo-N-[12-(1H-imidazol-1-yl) dodecyl]benz[cd]indol-2-amine | 90 |
| 6-Bromo-N-[4-(3-pyridinyl)butyl]benz-[cd]indol-2-amine sesqui-fumarate | 92 |
| 6-Bromo-N-[4-(3-pyridinyl)butyl]benz-[cd]indol-2-amine, dihydrochloride | 89 |

The novel compounds of the present invention have been found to be highly useful for inhibiting thromboxane synthetase in mammals when administered in amounts ranging from 1.0 mg to 20.0 mg/kg of body weight per day. A preferred dosage regimen for optimum results would be from 1.0 mg to 10.0 mg/kg of body weight per day. Such dosage units are employed that a total of from 70 to 700 mg of active compound for a subject of about 70 kg of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. The compounds of this invention are preferably administered orally but may be administered in any convenient manner such as by the intravenous, intramuscular, or subcutaneous routes.

Hypotensive Activity in Spontaneously Hypertensive Rats

The novel compounds of the present invention are active hypotensive agents and were tested for hypotensive activity by the method of P.S. Chan and D. Poorvin, Clinical and Experimental Hypertension, 1 (6), 817-830 (1979). Male, 16 week old, spontaneously hypertensive rats of the Okamoto strain, from Taconic Farms, Germantown, New York, having an average mean arterial blood pressure of 160±1.5 mm of mercury, were used in the test. One to 4 rats were used per test compound. A rat was dosed by gavage with a test compound, suspended in 2% pre-boiled starch at a concentration of 50 mg/ml, at a dose of 100 mg/kg of body weight or less, with 0.9% sodium chloride loading at a dose of 25 ml/kg of body weight. A second identical dose of the test compound, without sodium chloride loading was given 24 hours later. At 28 hours after initial dose the mean arterial blood pressure (MABP) was measured. The procedure was repeated in a second and third rat when necessary.

The results of this test on representative compounds of the present invention appear below in Table II.

TABLE II

Hypotensive Activity

| Compound | Avg. MABP in mm Hg (No. of Rats) (x 1.33 mbar) |
|---|---|
| N-[3-(1H-Imidazol-1-yl)propyl]benz[cd]-indol-2-amine, dihydrochloride | 87(2) |
| 6-Bromo-N-[3-(1H-imidazol-1-yl)propyl]-benz[cd]indol-2-amine, dihydrochloride | 117(2) |
| N-[3-(1H-Imidazol-1-yl)butyl]benz[cd]-indol-2-amine, dihydrochloride | 104(2) |
| N-[3-(4-Methyl-1H-imidazol-1-yl)propyl]-benz[cd]indol-2-amine, dihydrochloride | 106(2) |
| N-[3-(1H-Imidazol-1-yl)-2-methylpropyl]-benz[cd]indol-2-amine, dihydrochloride | 113(2) |
| N-[5-(1H-Imidazol-1-yl)pentyl]benz[cd]-indol-2-amine, fumarate | 111(2) |
| (Z)-N-[4-(1H-Imidazol-1-yl)-2-butenyl]-benz[cd]indol-2-amine, dihydrochloride | 77(2) |
| N-[3-(1H-imidazol-1-yl)-2,2-diphenyl-propyl]benz[cd]indol-2-amine | 127(3) |
| (Z)-N-[4-(1H-Imidazol-1-yl)-2-butenyl]-benz[cd]indol-2-amine, fumarate | 102(1) |
| N-[4-(1H-Imidazol-1-yl)butyl]benz[cd]-indol-2-amine, hydriodide | 74(2) |
| (Z)-N-[4-(1H-Imidazol-1-yl)-2-butenyl]-benz[cd]indol-2-amine, hydriodide | 106(2) |
| N-[3-(1H-Benzimidazol-1-yl)propyl]benz-[cd]indol-2-amine, fumarate | 123(3) |
| N-[3-(1H-Benzimidazol-1-yl)propyl]benz-[cd]indol-2-amine | 124(3) |
| N-[4-(1H-Imidazol-1-yl)butyl]benz[cd]-indol-2-amine, fumarate | 106(2) |
| N-[5-(1H-Imidazol-1-yl)-3-methylpentyl]-benz[cd]indol-2-amine, dihydrochloride | 92(2) |

TABLE II (Continued)

| Compound | Avg. MABP in mm Hg (No. of Rats) (x 1.33 mbar) |
|---|---|
| N-[10-(1H-Imidazol-1-yl)decyl]benz[cd]-indol-2-amine, fumarate | 124(3) |
| N-[10-(1H-Imidazol-1-yl)decyl]benz[cd]-indol-2-amine, dihydrochloride | 124(3) |
| N-[2-(1H-Imidazol-1-yl)ethyl]benz[cd]-indol-2-amine | 100(2) |
| N-{2-[2-(1H-Imidazol-1-yl)ethoxy]ethyl}-benz[cd]indol-2-amine, fumarate | 108(2) |
| N-[8-(1H-Imidazol-1-yl)octyl]benz[cd]-indol-2-amine, dihydrochloride | 111(2) |
| N-[2-(1H-Imidazol-1-yl)ethyl]benz[cd]-indol-2-amine, fumarate | 97(2) |
| 6-Bromo-N-[3-(1H-Imidazol-1-yl)butyl]-benz[cd]indol-2-amine | 120(2) |
| 6-Bromo-N-[4-(1H-Imidazol-1-yl)butyl]-benz[cd]indol-2-amine | 120(2) |
| N-{[4-(1H-Imidazol-1-ylmethyl)phenyl]-methyl}benz[cd]indol-2-amine, fumarate | 121(3) |
| 6,8-Dichloro-N-[10-(1H-imidazol-1-yl)-decyl]benz[cd]indol-2-amine | 121(2) |
| 6,8-Dichloro-N-[3-(1H-imidazol-1-yl)-butyl]benz[cd]indol-2-amine | 102(2) |
| 6,8-Dichloro-N-[3-(1H-imidazol-1-yl)-propyl]benz[cd]indol-2-amine | 102(2) |
| 6,8-Dichloro-N-[4-(1H-imidazol-1-yl)-butyl]benz[cd]indol-2-amine | 115(2) |
| 6-Bromo-N-[5-(1H-imidazol-1-yl)pentyl]-benz[cd]indol-2-amine | 118(3) |
| 6,8-Dichloro-N-[5-(1H-imidazol-1-yl)-pentyl]benz[cd]indol-2-amine | 119(2) |

TABLE II (Continued)

| Compound | Avg. MABP in mm Hg (No. of Rats) (x 1.33 mbar) |
|---|---|
| N-[4-(1H-imidazol-1-yl)pentyl]benz-[cd]indol-2-amine, fumarate | 97(2) |
| 6-Chloro-N-[4-(1H-imidazol-1-yl)-butyl]benz[cd]indol-2-amine, fumarate | 115(2) |
| 6-Chloro-N-[3-(1H-imidazol-1-yl)-propyl]benz[cd]indol-2-amine | 124(4) |
| N-[12-(1H-imidazol-1-yl)dodecyl]benz-[cd]indol-2-amine | 133(2) |
| 6-Bromo-N-[12-(1H-imidazol-1-yl)-dodecyl]benz[cd]indol-2-amine | 128(3) |
| N-Benz[cd]indol-2-yl-N-[4-(1H-imidazol-1-yl)butyl]acetamide | 81(2) |
| N-[3-(1H-Imidazol-1-yl)propyl]benz-[cd]indol-2-amine, bis-fumarate | 81(2) |
| N-[3-(1H-Imidazol-1-yl)propyl]-N-methylbenz[cd]indol-2-amine | 95(3) |
| N-[7-(1H-Imidazol-1-yl)heptyl]benz-[cd]indol-2-amine | 127(2) |

TABLE II (Continued)

| Compound | Avg. MABP in mm Hg (No. of Rats) (x 1.33 mbar) |
|---|---|
| 6,8-Dichloro-N-[3-(1H-Imidazol-1-yl)-butyl]benz[cd]indol-2-amine dihydrochloride | 134(3) |
| 6,8-Dichloro-N-[3-(1H-Imidazol-1-yl)-2-methylpropyl]benz[cd]indol-2-amine sesqui-fumarate | 116(2) |
| 6,8-Dichloro-N-[7-(1H-Imidazol-1-yl)-heptyl]benz[cd]indol-2-amine dihydrochloride | 130(2) |
| N-{[2-Ethyl-2-(1H-Imidazol-1-yl)methyl]butyl}benz[cd]indol-2-amine, sesqui-fumarate | 112(2) |
| 6-Bromo-N-[3-(1H-imidazol-1-yl)-2-methylpropyl]benz[cd]indol-2-amine, dihydrochloride | 107(2) |
| 6-Fluoro-N-[3-(1H-imidazol-1-yl)-propyl]benz[cd]indol-2-amine | 94(2) |
| N-[4-(1H-Imidazol-1-yl)butyl]]benz[cd]-indol-2-amine, dimethiodide | 110(2) |
| 6-Bromo-N-{4-[3-(pyridinyl)butyl]benz-[cd]indol-2-amine, sesqui-fumarate | 110(2) |
| 6-Bromo-N-[4-(3-pyridinyl)butyl]benz-[cd]indol-2-amine dihydrochloride | 99(2) |
| N-[2-(1H-Imidazol-1-yl)ethyl]benz-[cd]indol-2-amine | 101(2) |

The compounds of this invention are also considered to be cardio-protective in that they are anti-arrhythmic agents as established in the following test.

Thevetin (Cardiac Glycoside)-Induced Arrhythmia in Guinea Pigs

Male guinea pigs, weighing 300-500 g each, from Summit View Farms, Belvidere, N. J., were anesthetized by intraperitoneal administration of urethan at 1500 mg/kg. The animals were then restrained in a supine position. Electrocardiogram leads were attached to the four limbs and Lead II of the electrocardiogram was monitored.

The neck region was exposed and the jugular vein was cannulated. The test compounds were dissolved in saline and administered intravenously at the indicated doses, via a cannula which was then flushed with saline. Five minutes after the test compound was administered, thevetin dissolved in saline was administered by infusion through a cannula at a dose of 0.1 mg/kg/minute in a volume of 0.1 ml. The time until the P wave of the electrocardiogram disappeared was determined.

Based on the data obtained from 126 guinea pigs treated with physiological saline, but no test compound, the time it took for thevetin infusion to cause P wave disappearance on the electrocardiogram or the appearance of irregular heart beat (ectopic heart beat, etc.) was 22.91±0.5 minutes (mean±S.E.M).

A compound that protects guinea pigs for 31 minutes before arrhythmia occurred is considered active.

Propanolol at an intravenous dose of 2 mg/kg protected the guinea pigs for 47.0±4.1 minutes before arrhythmia occurred and was active in this test.

4-[2-(1H-Imidazol-1-yl)ethoxy]benzoic acid, monohydrochloride (Dazoxiben hydrochloride, Pfizer, Inc.) and 2-methyl-3-[4-(3-pyridinylmethyl)phenyl]-2-propenoic acid, sodium salt (OKY-1581, Ono Pharm.), both literature-described thromboxane synthetase inhibitors, at intravenous doses as high as 20 mg/kg were inactive (22.2±1.9 and 24.3±2.2 minutes, respectively).

The results of this test on typical compounds of this invention appear in Table III.

TABLE III

| Compound | No. of Guinea Pigs | IV Dose (mg/kg) | Time (Minutes) Before Induced Arrhythmia |
|---|---|---|---|
| N-[3-(1H-Imidazol-1-yl)propyl]benz[cd]indol-2-amine, dihydrochloride | 4 | 10 | 48.5 |
| N-[3-(1H-Imidazol-1-yl)-2-methylpropyl]-benz[cd]indol-2-amine, fumarate | 4 | 20 | 34.5 |
| N-[3-(1H-Imidazol-1-yl)-2,2-diphenylpropyl]-benz[cd]indol-2-amine | 4 | 10 | 41 |
| N-[4-(1H-Imidazol-1-yl)butyl]benz[cd]indol-2-amine, fumarate | 6 | 10 | 31 |
| N-[10-(1H-Imidazol-1-yl)decyl]benz[cd]indol-2-amine, fumarate | 6 | 10 | 34.2 |
| 6-Bromo-N-[3-(1H-imidazol-1-yl)butyl]benz[cd]-indol-2-amine | 3 | 30 | 34.7 |
| 6,8-Dichloro-N-[3-(1H-imidazol-1-yl)butyl]-benz[cd]indol-2-amine | 6 | 20 | 32 |
| 6,8-Dichloro-N-[5-(1H-imidazol-1-yl)pentyl]-benz[cd]indol-2-amine | 5 | 20 | 33.4 |
| 6-Chloro-N-4-[(1H-imidazol-1-yl)butyl]benz[cd]-indol-2-amine, fumarate | 2 | 10 | 31.5 |

TABLE III (Continued)

| Compound | No. of Guinea Pigs | IV Dose (mg/kg) | Time (Minutes) Before Induced Arrhythmia |
|---|---|---|---|
| 6-Bromo-N-[5-(1H-imidazol-1-yl)pentyl]benz[cd]-indol-2-amine | 2 | 20 | 37 |
| 2-{[3-(1H-Imidazol-1-yl)butyl]amino}-N,N-dimethylbenz[cd]indole-6-sulfonamide | 9 | 10 | 33.6 |
| N-[3-(1H-Imidazol-1-yl)-1-phenylpropyl]benz-[cd]indol-2-amine, fumarate | 8 | 30 | 34 |
| (E)-N-[4-(1H-Imidazol-1-yl)-2-butenyl]benz[cd]-indol-2-amine, monohydriodide | 3 | 10 | 34.3 |
| 2-{[3-(1H-Imidazol-1-yl)propyl]amino}-N,N'-dimethylbenz[cd]indole-6-sulfonamide | 5 | 20 | 33.4 |
| N-[2-(3-Pyridinyl)ethyl]benz[cd]indol-2-amine sesqui-fumarate | 2 | 20 | 37 |

It is known that drugs that have alpha-adrenoceptor binding activity are capable of blocking alpha-adrenoceptors on the heart muscle and are thus implicated in the prevention of several injuries that are associated with myocardial infraction.

## In Vitro Test for Alpha-Adrenoceptor Binding in Heart Membrane

Mycardial membrane protein was isolated from Sprague-Dawley rats by an art recognized method. Each test compound was then incubated at a concentration of 10 micro-moles, in the presence of a known amount of membrane (about 500 micro-grams) and a radioactive ligand, $3_H$-prazocin. Displacement of the ligand by the test compound was then calculated by assessing the amount of radioactivity associated with membrane using a liquid scintillation counter. Specific binding of 65% or more of the total radioactivity to the membrane in the presence of the test compound is the criterion for designating a particular compound as "in vitro active". The results of this test appear in the following Table.

In Vitro Results for Alpha-Adrenoceptor Binding in Heart Membrane

| Compound | Percent* Specific Binding |
|---|---|
| N-[3-(2-Methyl-1H-imidazol-1-yl)propyl]benz[cd]indol-2-amine, monohydriodide | 66.7 |
| N-[4-(1H-Imidazol-1-yl)butyl]benz[cd]indol-2-amine, fumarate | 78.4 |
| N-[10-(1H-Imidazol-1-yl)decyl]benz[cd]indol-2-amine, fumarate | 64.8 |
| N-[10-(1H-Imidazol-1-yl)decyl]benz[cd]indol-2-amine, dihydrochloride | 87.1 |
| N-[4-(1H-Imidazol-1-yl)pentyl]benz[cd]indol-2-amine, fumarate | 65.3 |
| 6-Chloro-N-[4-(1H-imidazol-1-yl)butyl]benz[cd]indol-2-amine, fumarate | 65.6 |
| N-[3-(1H-Imidazol-1-yl)propyl]benz[cd]indol-2-amine, difumarate | 71.7 |
| N-[3-(1H-Imidazol-1-yl)-2-methyl]propylbenz[cd]indol-2-amine fumarate | 67.0 |
| 6-Bromo-N-[10-(1H-imidazol-yl)decyl]benz[cd]indol-2-amine | 66.7 |
| 6-Bromo-N-[4-(3-pyridinyl)butyl]benz[cd]indol-2-amine fumarate | 68.4 |

*Mean of three separate incubations containing test compound at 10 micro-moles and 2.5 nM of H-prazocin.

Compositions according to the present invention having the desired clarity, stability and adaptability for parenteral use are obtained by dissolving from 0.10% to 10.0% by weight of active compound in a vehicle consisting of a polyhydric aliphatic alcohol or mixtures thereof. Especially satisfactory are glycerin, propylene glycol, and polyethylene glycols. The polyethylene glycols consist of a mixture of non-volatile, normally liquid, polyethylene glycols which are soluble in both water and organic liquids and which have a molecular weight of from about 200 to 1500. Although the amount of active compound dissolved in the above vehicle may vary from 0.10% to 10.0% by weight, it is preferred that the amount of active compound employed be from 3.0 to 9.0% by weight. Although various mixtures of the aforementioned non-volatile polyethylene glycols may be employed, it is preferred to use a mixture having an average molecular weight of from 200 to 400.

In addition to the active compound, the parenteral solutions may also contain various preservatives which may be used to prevent bacterial and fungal contamination. The preservatives which may be used for these purposes are, for example, myristyl-gamma-picolinium chloride, benzalkonium chloride, phenethyl alcohol, p-chlorophenyl-glycerol ether- methyl and propyl parabens, and thimerosal. As a practical matter, it is also convenient to employ antioxidants. Suitable antioxidants include, for example, sodium bisulfite, sodium metabisulfite, and sodium formaldehyde sulfoxylate. Generally, from 0.05 to 0.2% concentrations of antioxidant are employed.

For intramuscular injection, the preferred concentration of active compound is 0.25 to 0.50 mg/ml of the finished compositions. The novel compounds of the present invention are equally adapted to intravenous administration when diluted with water or diluents employed in intravenous therapy such as isotonic glucose in appropriate quantities. For intravenous use, initial concentrations down to about 0.05 to 0.25 mg/ml of active ingredient are satisfactory.

The active compounds of the present invention may be orally administered, for example, with an inert diluent or with an assimilable edible carrier, or they may be enclosed in hard or soft shell gelatin capsules, or they may be compressed into tablets, or they may be incorporated directly with the food of the diet. For oral therapeutic administration, the active compounds may be incorporated with excipients and used in the form of tablets, troches, capsules, elixirs, suspensions, syrups, wafers, and the like. Such compositions and preparations should contain at least 0.1% of active compound. The percentage of the compositions and

preparations may, of course, be varied and may conveniently be between 2% to 60% of the weight of the unit. The amount of active compound in such therapeutically useful compositions is such that a suitable dosage will be obtained.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier such as a fatty oil. Various other materials may be present as coatings or to otherwise modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed.

The following specific examples illustrate the preparation of the compounds of the present invention.

## Example 1

### N-[3-(1H-Imidazol-1-yl)propyl]-benz[cd]indol-2-amine, dihydrochloride

A mixture of 6.2 g of benz[cd]indole-2-thiol and 4.4 g of 3-(1H-imidazol-1-yl)propanamine in 250 ml of ethanol was stirred and heated. An 8.0 g portion of mercuric oxide was added, the mixture was stirred at reflux for 20 hours, then filtered and the insolubles washed with 100 ml of hot ethanol. The combined filtrate and wash was taken to dryness in vacuo. The residual oil was dissolved in a mixture of 100 ml of water and 15 ml of concentrated hydrochloric acid, treated with activated charcoal and then filtered. The filtrate was taken to dryness in vacuo. The residual oil was mixed with 150 ml of ethanol and taken to dryness in vacuo. This residue was dissolved in 100 ml of boiling ethanol, then filtered and the filtrate cooled at -10°C. This filtrate was then reheated to boiling, 300 ml of acetone were added, the mixture treated with activated charcoal and then filtered. The filtrate was cooled at -10°C and the resulting precipitate collected, washed with acetone and dried in vacuo at 60°C, giving 3.4 g of the desired product, mp 262°C-265°C (dec.).

## Example 2

### 6-Bromo-N-[3-(1H-Imidazol-1-yl)propyl]-benz[cd]indol-2-amine, dihydrochloride

A mixture of 4.0 g of 6-bromo-2-benz[cd]indole-2-thiol and 2.0 g of 3-(1H-imidazol-1-yl)propanamine in 125 ml of 2-methoxyethanol was stirred and heated. A 3.8 g portion of mercuric oxide was added and the mixture was stirred at reflux for 7 hours, then clarified while hot. The filtrate was cooled to -10°C, acidified with 5 ml of concentrated hydrochloric acid and then taken to dryness in vacuo. The residue was dissolved in 150 ml of boiling ethanol, filtered, cooled to -10°C and 150 ml of acetone added. This mixture was allowed to stand at 10°C, then the precipitate was collected, washed with acetone and dried in vacuo at 60°C, giving 1.5 g of the desired product, mp 281°C-283°C (dec.).

**Example 3**

**N-[3-(1H-Imidazol-1-yl)butyl]-benz[cd]indol-2-amine, dihydrochloride**

A mixture of 2.8 g of 3-(1H-imidazol-1-yl)butanamine, 6.5 g of 2-(methylthio)benz[cd]indole hydriodide and 250 ml of ethanol was stirred at reflux for 16 hours, then 2 g of potassium carbonate and 10 ml of water were added and the mixture was taken to dryness in vacuo. The residue was partitioned between 250 ml of dichloromethane and 100 ml of water. The dichloromethane layer was separated, dried over magnesium sulfate, filtered and the filtrate taken to dryness in vacuo. The residue was mixed with 200 ml of 2-methoxyethanol and 5 ml of concentrated hydrochloric acid, then taken to dryness in vacuo. The residue was dissolved in 50 ml of hot ethanol, diluted with 200 ml of acetone, cooled to -10°C, diluted with 200 ml of ether and then filtered. The filtrate was diluted with 400 ml of ether and cooled at -10°C. The precipitate was collected, washed with ether and dried in vacuo at 60°C, giving 0.8 g of the desired product, mp 145°C-150°C (dec.).

**Example 4**

**N-[1-(4-Chlorophenyl)-2-(1H-Imidazol-1-yl)-ethyl]benz[cd]indol-2-amine, fumarate**

A mixture of 2 g of 1-(4-chlorophenyl)-2-(1H-imidazol-1-yl)ethanamine, 2.95 g of 2-(methylthio)benz[cd]-indole hydriodide and 200 ml of ethanol was reacted as described in Example 3. The resulting base was dissolved in 50 ml of acetone, filtered and the filtrate added to a solution of 0.3 g of fumaric acid in 50 ml of acetone. The mixture was cooled to -10°C, the solid collected, washed with acetone and ether and dried at 60°C in vacuo, giving 0.6 g of the desired product, mp 130°C-135°C (dec.).

**Example 5**

**N-[3-(4-Methyl-1H-Imidazol-1-yl)-propyl]benz[cd]indol-2-amine, dihydrochloride**

A 1.4 g portion of 3-(4-methyl-1H-imidazol-1-yl)propanamine, 1.9 g of benz[cd]indole-2-thiol, 250 ml of ethanol and 2.5 g of mercuric oxide were reacted as described in Example 1, giving 0.3 g of the desired product, mp 250°C-255°C (dec.).

**Example 6**

**N-[3-(1H-Imidazol-1-yl)-2-methylpropyl]benz[cd]indol-2-amine, dihydrochloride**

A mixture of 7 g of 2-methyl-3-(1H-imidazol-1-yl)propanamine, 9.3 g of benz[cd]indole-2-thiol, 300 ml of ethanol and 13 g of mercuric oxide was reacted as described in Example 1, giving 1.2 g of the desired product, mp 250°C-255°C (dec.).

**Example 7**

**N-[3-(1H-Imidazol-1-yl)-1-phenylpropyl]benz[cd]indol-2-amine, fumarate**

A mixture of 4 g of phenyl-3-(1H-imidazol-1-yl)propanamine, 3.7 g of benz[cd]indole-2-thiol, 250 ml of ethanol and 6 g of mercuric oxide was reacted as described in Example 1, giving the dihydrochloride salt of the desired product, which was then converted to the fumarate salt giving 0.7 g of the desired product, mp 125°C-127°C.

**Example 8**

**N-[3-(1H-Imidazol-1-yl)-2-methylpropyl]benz[cd]indol-2-amine, fumarate**

A portion of the corresponding dihydrochloride salt, prepared in Example 6, was reacted as described in Example 4, giving 0.7 g of the fumarate salt, mp 150°C-154°C.

**Example 9**

**N-[5-(1H-Imidazol-1-yl)-pentyl]benz[cd]indol-2-amine, fumarate**

A mixture of 1.55 g of 5-(1H-imidazol-1-yl)pentanamine, 3.3 g of 2-(methylthio)benz[cd]indole hydriodide, and 200 ml of ethanol was reacted as described in Example 3, giving 4.6 g of the corresponding dihydrochloride salt, which was further reacted as described in Example 4, giving 1.6 g of the desired product, mp 159°C-161°C (dec.).

**Example 10**

**(Z)-N-[4-(1H-Imidazol-1-yl)-2-butenyl]-benz[cd]indol-2-amine, dihydrochloride**

A mixture of 2.5 g of (Z)-(1H-imidazol-1-yl)2-butenamine, 5.9 g of 2-(methylthio)benz[cd]indole hydriodide, and 500 ml of ethanol was reacted as described in Example 3, giving 0.7 g of the desired product, mp 215°C-220°C (dec.).

**Example 11**

**N-[3-(1H-Imidazol-1-yl)-2,2-diphenylpropylbenz[cd]indol-2-amine**

A mixture of 3.3 g of 2-(methylthio)benz[cd]indole hydriodide, 1.8 g of 1H-imidazole-1-(2,2-diphenyl)-propanamine, 200 ml of ethanol and 0.9 g of sodium acetate was stirred at reflux for 18 hours, then diluted with 150 ml of water containing 1 g of sodium bicarbonate. This mixture was concentrated to turbidity and cooled at -10°C. The mixture was divided into two portions and each was extracted with two 200 ml portions of dichloromethane. All four extracts were combined, washed with 250 ml of water, dried over magnesium sulfate and filtered. The filtrate was evaporated at 40°C. The residual oil was extracted with two 100 ml portions of boiling hexane. The hexane was decanted, the residual solid washed with hexane, air dried and recrystallized from 200 ml of ethyl acetate, giving 1.3 g of the desired product, mp 229°C-230°C.

**Example 12**

**(Z)-N-[4-(1H-Imidazol-1-yl)-2-butenyl]benz[cd]-indol-2-amine and the corresponding fumarate salt**

A mixture of 3.3 g of 2-(methylthio)benz[cd]indole hydriodide, 2.2 g of (Z)-4-(1H-imidazol-1-yl)-2-butenamine, 200 ml of ethanol and 0.9 g of sodium acetate was reacted as described in Example 11, giving the desired base which was then converted to the corresponding fumarate salt as described in Example 4, giving 1.8 g of the desired product, mp 85°C-90°C (dec.).

**Example 13**

**N-[3-(2-Phenyl-1H-Imidazol-1-yl)-propyl]benz[cd]indol-2-amine, monohydriodide**

A mixture of 4.0 g of 3-(2-phenyl-1H-imidazol-1-yl)propanamine, 6.5 g of 2-(methylthio)benz[cd]indole hydriodide, 1.8 g of sodium acetate and 250 ml of ethanol was reacted as described in Example 11. The crude product was recrystallized from a mixture of ethanol and isopropanol giving 3.8 g of the desired product, mp 153°C-155°C.

**Example 14**

**N-[3-(2-Methyl-1H-Imidazol-1-yl)-propyl]benz[cd]indol-2-amine, monohydriodide**

A mixture of 2.1 g of 3-(2-methyl-1H-imidazol-1-yl)propanamine, 5.0 g of 2-(methylthio)benz[cd]indole hydriodide, 1.4 g of sodium acetate and 250 ml of ethanol was reacted as described in Example 13, giving 1.0 g of the desired product, mp 153°C-155°C.

**Example 15**

## N-[4-(1H-Imidazol-1-yl)-butyl]benz[cd]indol-2-amine, monohydriodide

A 2.2 g portion 4(1H-imidazol-1-yl)butanamine dihydrochloride and 2 ml of 10N sodium hydroxide in 200 ml of ethanol was stirred for 10 minutes and then treated with 3.2 g of 2-(methylthio)benz[cd]indole hydriodide. This mixture was heated at reflux for 16 hours and then cooled to -10°C. The mixture was reheated to boiling, clarified while hot and the filtrate cooled to -10°C. The solid was collected, washed with ethanol, then ether and dried at 60°C in vacuo, giving 2.0 g of the desired product, mp 144°C-147°C.

**Example 16**

## (E)-N-[4-(1H-Imidazol-1-yl)-2-butenyl]benz[cd]indol-2-amine, monohydriodide

A mixture of 5.0 g of (E)-4-(1H-imidazol-1-yl)-2-butenamine, 11.5 g of 2-(methylthio)benz[cd]indole hydriodide and 400 ml of ethanol was stirred and heated at reflux for 16 hours, then clarified while hot. The filtrate was concentrated to 250 ml, cooled to -10°C and the resulting solid collected, washed with ethanol, ether and dried at 60°C in vacuo, giving 9.3 g of the desired product, mp 152°C-155°C (dec.).

**Example 17**

## N-[3-(1H-Benzimidazol-1-yl)-propyl]benz[cd]indol-2-amine and fumarate salt

A mixture of 2.6 g of 3-(1H-benzimidazol-1-yl)propanamine, 2.8 g of benz[cd]-indole-2-thiol, 3.8 g of mercuric oxide and 250 ml of ethanol was reacted as described in Example 1, giving 1.5 g of the desired base mp 191°C-193°C, which was then converted to the fumarate salt as described in Example 4, giving 1.3 g of the desired product as fumarate salt, mp 155°C-158°C.

**Example 18**

## N-[4-(1H-Imidazol-1-yl)-butyl]benz[cd]indol-2-amine, fumarate

A 2.2 g portion of 4-(1H-imidazol-1-yl)butanamine dihydrochloride and 2 ml of 10N sodium hydroxide in 200 ml of ethanol was stirred for 10 minutes and then treated with 3.2 g of 2-(methylthio)benz[cd]indole hydriodide. This mixture was heated at reflux for 16 hours, and then taken to dryness in vacuo. The residue was partitioned between 250 ml of dichloromethane and 100 ml of 1N sodium hydroxide. The dichloromethane layer was dried over magnesium sulfate, filtered and the filtrate evaporated giving the crude base derivative. This base was treated with 1.5 g of fumaric acid in 400 ml of acetone, giving 2.4 g of the desired product, mp 153°C-155°C (dec.).

**Example 19**

**N-[5-(1H-Imidazol-1-yl)-3-methylpentyl]-benz[cd]indol-2-amine, dihydrochloride**

A 2.9 g portion of 5-(1H-imidazol-1-yl)-3-methylpentanamine in 350 ml of ethanol was treated with 5.5 g of 2-(methylthio)benz[cd]indole hydriodide and stirred at reflux for 18 hours. The mixture was concentrated to 175 ml, cooled to -10°C and clarified. The filtrate was taken to dryness in vacuo and the residue partitioned between 250 ml of dichloromethane and 100 ml of 1N sodium hydroxide. The dichloromethane layer was dried over magnesium sulfate, clarified and evaporated to dryness. The residue was dissolved in 400 ml of acetone, treated with 10 ml of 3.5N hydrochloric acid in ethanol, then concentrated to 200 ml on a steam bath and diluted to turbidity with ether. The mixture was cooled to -10°C and the solid collected, washed with acetone and dried in vacuo at 60°C, giving 1.5 g of the desired product, mp 113°C-116°C (dec.).

**Example 20**

**N-[10-(1H-Imidazol-1-yl)decyl]benz[cd]-indol-2-amine, fumarate and dihydrochloride**

A mixture of 6.6 g of 10-(1H-imidazol-1-yl)decanamine, 500 ml of ethanol and 9.8 g of 2-(methylthio)-benz[cd]indole hydriodide was reacted as described in Example 19, giving 9.7 g of the base form of the desired compound as a brown oil. A portion of this base was converted to the desired fumarate salt by the procedure described in Example 4, giving 1.1 g, mp 135°C-136°C.

A portion of the base derivative was converted to the dihydrochloride salt by treatment with hydrochloric acid in ethanol, giving 3.5 mp 103°C-105°C.

**Example 21**

**N-[2-(1H-Imidazol-1-yl)ethyl]benz[cd]-indol-2-amine, base and fumarate salt**

A mixture of 1.25 g of 2-(1H-imidazol-1-yl)ethanamine, 300 ml of ethanol, and 2.5 g of 2-(methylthio)-benz[cd]indole hydriodide was reacted as described in Example 19, giving 1.4 g of the base derivative, mp 172°C-173°C.

A portion of this base was then converted to the fumarate salt as described in Example 4, giving 1.0 g, mp 210°C-212°C.

**Example 22**

**N-{2-[2-(1H-Imidazol-1-yl)ethoxy]ethyl}-benz[cd]indol-2-amine, fumarate**

A mixture of 1.7 g of 2-[2-(1H-imidazol-1-yl)ethoxyethanamine], 600 ml of ethanol, and 3.2 g of 2-(methylthio)benz[cd]indole hydriodide was reacted as described in Example 19, giving the base derivative

which was then converted to the fumarate salt by the procedure of Example 4, giving 2.1 g, mp 153°C-154°C (dec.).

**Example 23**

**N-[8-(1H-Imidazol-1-yl)octyl]-benz[cd]indol-2-amine, dihydrochloride**

A mixture of 2.1 g of 8-(1H-imidazol-1-yl)octanamine, 400 ml of ethanol, and 3.2 g of 2-(methylthio)-benz[cd]indole hydriodide was reacted as described in Example 19, giving the base derivative which was then treated with hydrochloric acid giving 1.3 g of the desired hydrochloride salt, mp 222°C-224°C.

**Example 24**

**2-(Benz[cd]indole-2-ylamino)-N-[3-(1H-Imidazol-1-yl)propyl]**

A mixture of 3.7 g of N-[3-(1N-imidazol-1-yl)propyl]glycinamide, 425 ml of ethanol, and 5.0 g of 2-(methylthio)benz[cd]indole hydriodide was reacted as described in Example 19, giving 2.2 g of the desired product, mp 145°C-146°C.

**Example 25**

**N-{[4-(1H-Imidazol-1-ylmethyl)phenyl]-methyl}benz[cd]indol-2-amine, fumarate**

A mixture of 2.8 g of 4-(1H-imidazol-1-yl)methylbenzylamine, 250 ml of ethanol and 4.7 g of 2-(methylthio)benz[cd]indole hydriodide was reacted as described in Example 19 giving the base derivative which was then reacted as described in Example 4, giving 6.6 g of the fumarate salt, mp 200°C-205°C.

**Example 26**

**N-[4-(1H-Imidazol-1-yl)pentyl]-benz[cd]-2-amine, fumarate**

A mixture of 2.8 g of 4-(1H-imidazol-1-yl)pentanamine, 3.3 g of benz[cd]indole-2-thiol, 7.0 g of mercuric acetate and 500 ml of ethanol was reacted as described in Example 1, giving the base derivative which was converted to the fumarate salt as described in Example 4, giving 5.6 g, mp 173°C-175°C.

**Example 27**

**6-Bromo-N-[3-(1H-imidazol-1-yl)-butyl]benz[cd]indol-2-amine**

A mixture of 5.2 g of 6-bromo-benz[cd]indole-2-thiol, 2.8 g of 3-(1H-imidazol-1-yl)butanamine, 6.6 g of mercuric acetate and 100 ml of dry p-dioxane was reacted as described in Example 1, giving 3.0 g of the desired product, mp 240°C-241°C.

**Example 28**

**2-{[3-(1H-Imidazol-1-yl)butyl]-amino}N,N-dimethylbenz[cd]indol-6-sulfonamide**

A mixture of 7.3 g of 1,2-dihydro-N,N-dimethyl-2-thioxobenz[cd]indole-6-sulfonamide, 4.2 g of 3-(1H-imidazol-1-yl)butanamine, 150 ml of ethanol and 9.6 g of mercuric acetate was reacted as described in Example 1, giving 5.5 g of the desired product, mp 221°C-222°C.

**Example 29**

**2-{[3-(1H-Imidazol-1-yl)propyl]-amino}N,N-dimethylbenz[cd]indol-6-sulfonamide**

A mixture of 5.0 g of 1,2-dihydro-N,N-dimethyl-2-thioxobenz[cd]indole-6-sulfonamide, 2.3 g of 3-(1H-imidazol-1-yl)propanamine, 100 ml of ethanol and 5.4 g of mercuric acetate was reacted as described in Example 1, giving 2.7 g of the desired product, mp 199°C-201°C.

**Example 30**

**6-Bromo-N-[10-(1H-imidazol-1-yl)-decyl]benz[cd]indol-2-amine**

A mixture of 2.8 g of 10-(1H-imidazol-1-yl)decanamine, dihydrochloride was treated with 2 ml of 10N sodium hydroxide in an ethanol-water mixture giving the base derivative. To this base was added 25 ml of ethanol, 2.33 g of 6-bromo-benz[cd]indole-2-thiol and 3.0 g of mercuric acetate. The procedure of Example 1 was then followed, giving 2.8 g of the desired product, mp 115°C-116°C.

**Example 31**

**6-Bromo-N-[4-(1H-imidazol-1-yl)-butyl]benz[cd]indol-2-amine**

A mixture of 4.2 g of 4-(1H-imidazol-1-yl)butanamine dihydrochloride in 75 ml of ethanol was treated with 2.2 g of potassium hydroxide and stirred for 18 hours. A 5.2 g portion of 6-bromo-benz[cd]indole-2-thiol and 6.4 g of mercuric acetate were added and the reaction proceeded as described in Example 1, giving 3.7 g of the desired product, mp 145°C-147°C.

**Example 32**

**6,8-Dichloro-N-[10-(1H-imidazol-1-yl)-decyl]benz[cd]indol-2-amine**

A 2.8 g portion of 10-(1H-imidazol-1-yl)decanamine, dihydrochloride in an ethanol-water mixture was treated with 2.0 ml of 10N sodium hydroxide, stirred and evaporated to dryness. To the residue was added 35 ml of dry dimethylformamide, 3 g of mercuric acetate and 2.2 g of 6,8-dichloro-benz[cd]indole-2-thiol. The reaction proceeded as described in Example 1, giving 2.3 g of the desired product, mp 129°C-131°C.

**Example 33**

**6,8-Dichloro-N-[3-(1H-imidazol-1-yl)-butyl]benz[cd]indol-2-amine**

A mixture of 7.0 g of 6,8-dichloro-benz[cd]indole-2-thiol, 100 ml of ethanol, 9.5 g of mercuric acetate and 4.2 g of 4-(1H-imidazol-1-yl)-2-butanamine was reacted as described in Example 1, giving 1.7 g of the desired product, mp 244°C-246°C.

**Example 34**

**6,8-Dichloro-N-[3-(1H-imidazol-1-yl)-propyl]benz[cd]indol-2-amine**

A mixture of 5.0 g of 6,8-dichloro-benz[cd]indole-2-thiol, 35 ml of dimethylformamide, 2.6 g of 3-(1H-imidazol-yl)propanamine and 6.3 g of mercuric acetate was reacted as described in Example 1, giving 2.95 g of the desired product, mp 182°C-183°C.

**Example 35**

**6,8-Dichloro-N-[4-(1H-imidazol-1-yl)-butyl]benz[cd]indol-2-amine**

A 4.2 g portion of 4-(1H-imidazol-1-yl)butanamine, dihydrochloride was suspended in 75 ml of ethanol, treated with 2.24 g of potassium hydroxide, stirred for 6 hours and evaporated. A 35 ml portion of dimethylformamide, 5.0 g of 6,8-dichloro-benz[cd]indole-2-thiol, and 6.3 g of mercuric acetate were added and the reaction proceeded as described in Example 1, giving 2.5 g of the desired product, mp 187°C-188°C.

**Example 36**

**6-Bromo-N-[5-(1H-imidazol-1-yl)-pentyl]benz[cd]indol-2-amine**

A mixture of 5.3 g of 6-bromo-benz[cd]indole-2-thiol, 3.1 g of 5-(1H-imidazol-1-yl)pentanamine, 100 ml of ethanol and 6.3 g of mercuric acetate was reacted as described in Example 1, giving 1.5 g of the desired product, mp 138°C-140°C.

**Example 37**

**6,8-Dichloro-N-[5-(1H-imidazol-1-yl)-pentyl]benz[cd]indol-2-amine**

A mixture of 5.0 g of 6,8-dichloro-benz[cd]indole-2-thiol, 3.1 g of 5-(1H-imidazol-1-yl)pentanamine, 100 ml of dry dimethylformamide and 6.3 g of mercuric acetate was reacted as described in Example 1, giving 1.8 g of the desired product, mp 191°C-192.5°C.

**Example 38**

**N-[12-(1H-Imidazol-1-yl)-dodecyl]benz[cd]indol-2-amine**

A mixture of 2.5 g of 12-(1H-imidazol-1-yl)dodecanamine, 1.9 g of benz[cd]indole-2-thiol, 3.4 g of mercuric acetate and 400 ml of ethanol was reacted as described in Example 1. The crude product was purified by dissolving it in chloroform and chromatographing it on a silica gel column, eluting with 10% methanol in chloroform giving 490 mg of the desired product, mp 95°C-97°C.

**Example 39**

**6-Bromo-N-[12-(1H-imidazol-1-yl)-dodecyl]benz[cd]indol-2-amine**

The procedure of Example 38 was repeated using 2.65 g of 6-bromo-benz[cd]indole-2-thiol instead of 1.9 g of benz[cd]indole-2-thiol, giving 720 mg of the desired product, mp 111°C-116°C.

**Example 40**

**6-Chloro-N-[5-(1H-imidazol-1-yl)-pentyl]benz[cd]indol-2-amine**

A mixture of 4.4 g of 6-chloro-benz[cd]indole-2-thiol, 3.06 g of 5-(1H-imidazol-1-yl)pentanamine, 6.3 g of mercuric acetate and 150 ml of ethanol was reacted as described in Example 1, giving 5.2 g of the desired product, mp 132°C-134°C.

**Example 41**

**6-Chloro-N-[4-(1H-imidazol-1-yl)-butyl]benz[cd]indol-2-amine, fumarate**

A mixture of 4.4 g of 6-chloro-benz[cd]indole-2-thiol, 2.8 g of 4-(1H-imidazol-1-yl)butanamine, 6.3 g of mercuric acetate and 150 ml of ethanol was reacted as described in Example 1, giving the free base form. This free base was converted to the fumarate salt by the procedure of Example 4, giving 4.8 g mp 190°C-192°C.

**Example 42**

**6-Chloro-N-[3-(1H-imidazol-1-yl)-propyl]benz[cd]indol-2-amine**

A mixture of 1.0 g of 6-chloro-benz[cd]indole-2-thiol, 540 mg of 3-(1H-imidazol-1-yl)propanamine, 1.26 g of mercuric acetate and 100 ml of ethanol was reacted as described in Example 1, giving 1.0 g of the desired compound, mp 177°C-178°C.

**Example 43**

**N-[3-(1H-imidazol-1-yl)-propyl]benz[cd]indol-2-amine, difumarate**

The free base, N-[3-(1H-imidazol-1-yl)propyl]benz[cd]indole-2-amine (prepared as described in Example 1) was dissolved in 20 parts (by weight) of acetone and this solution added dropwise to a vigorously stirred, refluxing solution of 2.2 equivalents of fumaric acid in 150 parts of acetone. After cooling to room temperature, the bright yellow precipitate was collected, washed with acetone and dried in vacuo at 60°C, giving the desired fumarate salt, mp 165°C-166°C (dec.).

**Example 44**

**(Z)-N-[4-(1H-Imidazol-1-yl)-2-butenyl]benz[cd]indol-2-amine difumarate**

The free base of the product of Example 10 was dissolved in acetone and added to a boiling solution of two equivalents of fumaric acid in acid. After cooling to room temperature, the bright yellow precipitate of the desired product was collected and dried. It melted at 111°C-113°C.

**Example 45**

**N-(2-Pyridinylmethyl)-benz[cd]indol-2-amine fumarate**

(not claimed)

A mixture consisting of 4.9 grams of 2-methylthiobenz[cd]indole hydriodide, 1.7 grams of 2-pyridinylmethylamine, and 100 ml of ethanol was stirred and heated under reflux for 16 hours. After concentration to dryness in vacuo, the residue was partitioned between 250 ml of dichloromethane and 100 ml of 1N NaOH solution. The dichloromethane layer was dried over $MgSO_4$, and the dichloromethane removed in vacuo. The residual yellow-brown oil was dissolved in 100 ml of acetone and added to a stirred boiling solution of 3.5 grams of fumaric acid in 800 ml of acetone. A precipitate formed immediately. After cooling to room temperature, the precipitate was collected, washed with acetone and dried. The yield of desired product was 3.4 grams; mp 210°C-213°C with decomposition.

**Example 46**

**N-(3-Pyridinylmethyl)-benz[cd]indol-2-amine fumarate**

(not claimed)

A mixture consisting of 3.7 grams of benz[cd]indole-2-thiol, 2.3 grams of 3-pyridinylmethylamine, and 300 ml of ethanol was stirred as 6.6 grams of mercuric acetate was added. The mixture was then stirred and heated under reflux for 16 hours, the slurry changing slowly from a yellow-brown color to a deep black. The hot mixture was filtered through diatomaceous earth; the black precipitate was washed with 100 ml of ethanol. After addition of 5 ml of 10N NaOH solution, the reaction mixture was taken to dryness in vacuo.

The residue was partitioned between 250 ml dichloromethane and 100 ml of water. The dichloromethane layer was dried over $MgSO_4$, and the dichloromethane removed in vacuo. The residual orange-yellow oil was dissolved in 100 ml of acetone, and added to a stirred boiling solution of 5 grams of fumaric acid in 1200 ml of acetone. A heavy yellow precipitate formed at once. After cooling to room temperature, the precipitate was collected, washed with acetone and dried. The yield of subject compound was 5.9 g and the melting point was 192°C-194°C with decomposition.

**Example 47**

**6-Bromo-N-[4-(3-pyridinyl)butyl]-benz[cd]indol-2-amine sesqui-fumarate**

4-(3-Pyridinyl)butylamine was prepared by the procedure described in Helv. chim. acta 65 1868-1883 (1982). 6-Bromobenz[cd]indol-2-thiol was prepared by the action of $P_2S_5$ upon 6-bromobenz[cd]indol-2-one in refluxing pyridine.

A mixture consisting of 2.6 grams of 6-bromobenz[cd]indol-2-thiol, 1.5 grams of 4-(3-pyridinyl)-butylamine, and 250 ml of ethanol was stirred as 3.5 grams of mercuric acetate was added. The mixture was stirred and heated under reflux for 16 hours, a deep black slurry being formed. Twenty five ml of 1N NaOH solution was added, and the insolubles filtered off. The cake was washed with ethanol. The filtrate was taken to dryness in vacuo, and the residue then partitioned between 200 ml of dichloromethane and 100 ml of water. The dichloromethane was dried over $MgSO_4$, and the dichloromethane removed in vacuo. The residual orange oil was dissolved in 100 ml of acetone, and the solution added to a stirred boiling solution of 3.0 grams of fumaric acid in 800 ml of acetone. A yellow precipitate formed at once. After cooling to room temperature, the precipitate was collected, washed with acetone, and dried. The yield of desired compound was 2.6 grams, mp 138°C-140°C with decomposition.

**Example 48**

**N-[7-(1H-Imidazol-1-yl)-heptyl]benz[cd]indol-2-amine**

A mixture consisting of 5.0 grams of 2-methylthiobenz[cd]indol-2-amine hydriodide, 2.8 grams of 7-(1H-imidazol-1yl)heptylamine, and 75 ml of ethanol as stirred and heated under reflux for 16 hours. After addition of 20 ml of 1N NaOH solution, the reaction mixture was taken to dryness in vacuo. The residue was partitioned between 150 ml of dichloromethane and 100 ml of water. A solid appeared at the interface. It was collected by filtration, added to the dichloromethane filtrate, and the mixture taken to dryness in vacuo. The residual orange oil was dissolved in 100 ml of acetone and added to a stirred boiling solution of 3.5 grams of fumaric acid in 800 ml of acetone. The resultant yellow precipitate was collected, washed with acetone, and dissolved in 200 ml of hot water. The solution was clarified by treatment with activated charcoal. The clear aqueous solution was cooled and made basic with 0.5 N NaOH solution. The resultant organe precipitate was collected, washed with water and dried in vacuo at room temperature. The yield of the title compound was 2.2 grams, and the mp was 48°C-50°C.

**Example 49**

**N-[2-(1H-Imidazol-4-yl)-ethyl]benz[cd]indol-2-amine**

The preparation of the subject compound was carried out by the procedure of Example 45, utilizing 1.7 grams of 2-(1H-imidazol-4-yl)ethanamine in place of 2-pyridinylmethylamine. The crude reaction product was purified by recrystallization from 150 ml of 33% ethanol; yield, 1.4 grams; mp 255°C-257°C dec.

**Example 50**

**6,8-Dichloro-N-[3-(1H-imidazol-1-yl)-butyl]benz[cd]indol-2-amine dihydrochloride**

The product of Example 33 (0.53 gram) was dissolved in 150 ml of ethanol, and 1.5 ml of 2.3N ethanolic hydrogen chloride. The solution was concentrated to about 50 ml volume and diluted with 100 ml of diethyl ether. The precipitate of the title compound was collected, washed with diethyl ether, and dried; yield, 0.49 gram; mp 257°C-259°C with decomposition.

**Example 51**

**N-[2-Ethyl-2-(1H-imidazol-1-yl)methyl]-butylbenz[cd]indol-2-amine sesqui-fumarate**

The subject compound was prepared by the method of Example 46, employing 0.75 grams of 2-ethyl-2-(1H-imidazol-1-yl)-1-ylmethyl)butanamine, 0.84 grams of benz[cd]indol-2-thiol, 1.43 grams of mercuric acetate, and 100 ml of ethanol. The yield was 1.70 grams, and the mp was 179°C-181°C with decomposition.

**Example 52**

**6,8-Dichloro-N-[3-(1H-imidazol-1-yl)-2-methylpropyl]-benz[cd]indol-2-amine and its sesqui-fumarate**

The title compound was prepared by the method of Example 46 utilizing 7.6 grams of 6,8-dichlorobenz-[cd]indol-2-thiol, 4.2 grams of 3-(1H-imidazol)-2-methylpropanamine, 9.5 grams of mercuric acetate, and 300 ml of ethanol. The crude product was triturated with acetone and the mixture cooled at 5°C. The crystals of free base were collected, washed with diethyl ether and dried; mp 180°C-181°C.

A solution of 1.5 grams of the above free base in 100 ml of hot acetone was added to a stirred solution of 1.0 gram of fumaric acid in 100 ml of hot acetone. Orange crystals of the sesqui-fumarate salt were collected, washed with acetone and dried; yield, 2.1 grams; mp 131°C-132°C.

**Example 53**

**6,8-Dichloro-N-[7-(1H-imidazol-1-ylheptyl]benz[cd]indol-2-amine dihydrochloride**

The free base of the title compound was prepared by the method of Example 46, utilizing 5.4 grams of 7-(1H-imidazol-1-yl)heptanamine, 7.6 grams of 6,8-dichlorobenz[cd]indol-2-thiol, 9.5 grams of mercuric acetate, and 500 ml of ethanol. A solution of 2.5 grams of the free base in 100 ml warm ethanol was treated with 30 ml of 2.3N ethanolic hydrogen chloride. The resultant mixture was taken to dryness in vacuo, and the residual syrup boiled up in 400 ml of acetone. Cooling at room temperature gave 1.9 gram of the title compound, mp 198°C-201°C.

**Example 54**

**6-Bromo-N-[3-(1H-imidazol-1-yl)-2-methylpropyl]benz[cd]indol-2-amine dihydrochloride**

The free base of the title compound was prepared by the procedure of Example 46, employing 2.8 grams of 3-(1H-imidazol-1-yl)-2-methylpropanamine, 5.3 grams of 6-bromobenz[cd]indol-2-thiol, 6.45 grams of mercuric acetate, and 500 ml of ethanol. The crude free base was purified by the chromatographic procedure of Example 46, 5.5 grams of product being obtained. This was converted to the dihydrochloride salt by treatment with excess 2.3N ethanolic hydrogenchloride, followed by precipitation with acetone. The yield was 1.2 grams and the mp 180°C with decomposition.

**Example 55**

**6-Bromo-N-[4-(3-pyridinyl)butyl]-benz[cd]indol-2-amine dihydrochloride**

The free base of the title compound was prepared by the procedure of Example 46, utilizing 3.0 grams of 4-(3-pyridyl)butanamine, 5.3 grams of 6-bromobenz[cd]indol-2-thiol, 6.4 grams of mercuric acetate, and 500 ml of ethanol. The crude free base was purified by the chromatographic procedure of Example 46. It was then dissolved in 100 ml of hot acetone and added to a mixture of 25 ml of 3.4N ethanolic hydrogen chloride and 50 ml of ethanol. Partial evaporation of the solution gave yellow crystals of the title compound; yield 6.5 grams; mp 241°C-244°C.

**Example 56**

**6-Fluoro-N-[1H-imidazol-1-yl)-propyl]benz[cd]indol-2-amine**

The title compound was prepared by the procedure of Example 46, utilizing 1.3 grams of 3-(1H-imidazol-1-yl)propanamine, 2.0 grams of 6-fluorobenz[cd]indol-2-thiol, 3.2 grams of mercuric acetate, and 50 ml of ethanol. Purification of the crude product by recrystallization from acetone gave 0.92 gram; mp 159°C-

160°C.

**Example 57**

**N-[3-1H-imidazol-1-yl)-1-methyl-propyl]benz[cd]indol-2-amine difumarate**

The subject compound was prepared by the method of Example 45, utilizing 4.5 grams of 3-(1H-imidazol-1-yl)-1-methylpropanamine, 6.0 grams of 2-methylthiobenz[cd]indole hydriodide, and 400 ml of ethanol. A portion of the isolated free base was converted to the difumarate salt which melted at 195°C-197°C with decomposition.

## Claims

**Claims for the following Contracting States : BE, CH, DE, FR, GB, IT, LI, NL, SE**

1. A compound having the formula:

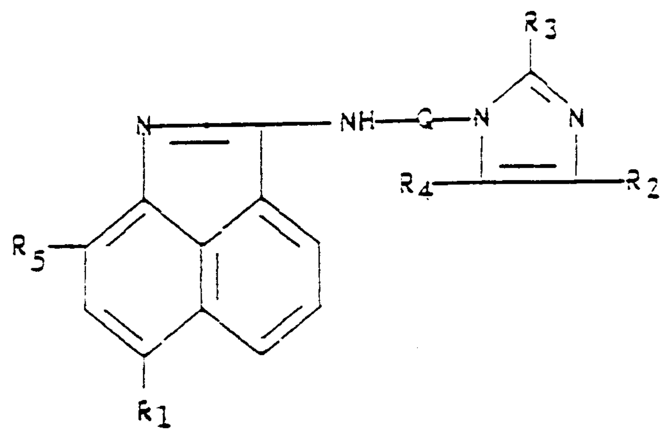

wherein $R_1$ is hydrogen, bromo, chloro or dimethylsulfonamide; $R_2$ is hydrogen or alkyl($C_1$-$C_3$); $R_3$ is hydrogen, alkyl($C_1$-$C_3$) or phenyl; $R_4$ is hydrogen or when taken together with $R_2$ is -CH=CH-CH=CH-; $R_5$ is hydrogen or chloro; and Q is $-(CH_2)_n-$, where n is an integer from 2 through 12,

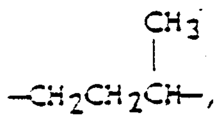
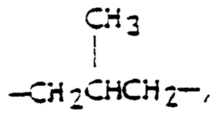
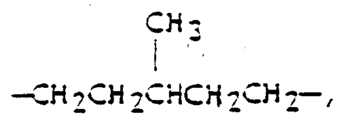
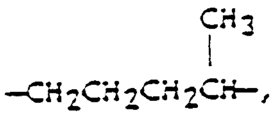
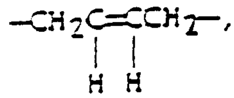
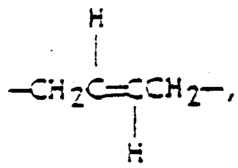
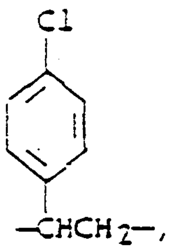
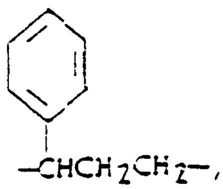
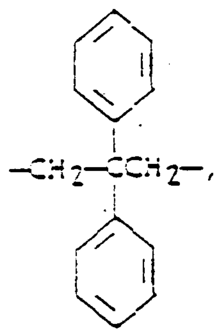
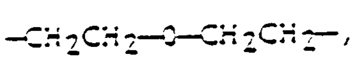
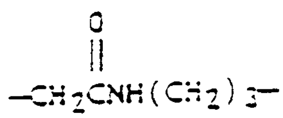
or
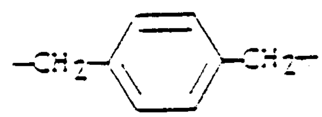

and the pharmacologically acceptable salts thereof.

2. The compound N-[3-(3-pyridinyl)propyl]benz[cd]indol-2-amine fumarate.

3. The compound N-[3-(3-pyridinyloxy)propyl]benz[cd]indol-2-amine fumarate.

4. The compound 6-bromo-N-[4-(3-pyridinyl)butyl]benz[cd]indol-2-amine sesqui-fumarate.

5. The compound N-[4-(3-pyridinyl)]benz[cd]indol-2-amine, hydriodide salt.

6. The compound N-[4-(3-pyridinyl)]benz[cd]indol-2-amine, sesquifumarate salt.

7. The compound N-[2-(3-Pyridinyl)ethyl]benz[cd]indol-2-amine, sesquifumarate.

8. The compound according to Claim 1, N-[4-(1H-imidazol-1-yl)butyl]benz[cd]indol-2-amine.

9. The compound according to Claim 1, N-[3-(1H-imidazol-1-yl)propyl]benz[cd]indol-2-amine.

**10.** The compound according to Claim 1, 6-bromo-N-[3-(1H-imidazol-1-yl)butyl]benz[cd]indol-2-amine.

**11.** The compound according to Claim 1, 6-bromo-N-[5-(1H-imidazol-1-yl)pentyl]benz[cd]indol-2-amine.

**12.** The compound according to Claim 1, 6,8-dichloro-N-[5-(1H-imidazol-1-yl)pentyl]benz[cd]indol-2-amine.

**13.** The compound according to Claim 1, 6-chloro-N-[3-(1H-imidazol-1-yl)butyl]benz[cd]indol-2-amine.

**14.** The compound according to Claim 1, N-[3-(1H-imidazol-1-yl)-1-phenylpropyl]benz[cd]indol-2-amine.

**15.** The compound according to Claim 1, N-[3-(1H-imidazol-1-yl)-2-methylpropyl]benz[cd]indol-2-amine.

**16.** The compound according to Claim 1, (E)-N-[4-(1H-imidazol-1-yl)-2-butenyl]benz[cd]indol-2-amine.

**17.** The compound according to Claim 1, 2-{[3-(1H-imidazol-1-yl)butyl]amino}-N,N-dimethylbenz[cd]indol-6-sulfonamide.

**18.** The compound according to Claim 1, 2-{[3-(1H-imidazol-1-yl)propyl]amino}-N,N-dimethylbenz[cd]indol-6-sulfonamide.

**19.** The compound according to Claim 1, 2-benz[cd]indole-2-ylamino)-N-[3-(1H-imidazol-1-yl)propyl]acetamide.

**20.** The compound according to Claim 1, (Z)-N-[4-(1H-imidazol-1-yl)-2-butanyl]benz[cd]indol2-amine.

**21.** The compound according to Claim 1, N-[5-(1H-imidazol-1-yl)-3-methylpentyl]benz[cd]indol-2-amine.

**22.** The compound according to Claim 1, 6,8-dichloro-N-[3-(1H-imidazol-1-yl)propyl]benz[cd]indol-2-amine.

**23.** A thromboxane synthetase enzyme inhibiting composition of matter in dosage unit form comprising from 10 mg to 700 mg of a compound of Claim 1 in association with a pharmacologically acceptable carrier.

**24.** A process for producing a compound according to Claim 1 of the formula:

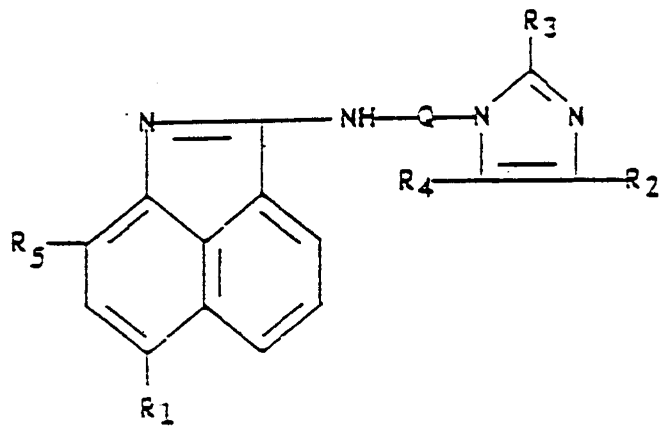

wherein $R_1$, $R_2$, $R_3$, $R_4$, $R_5$ and Q are as defined in Claim 1, and the pharmacologically acceptable salts which comprises reacting a substituted benz[cd]indol-2-thiol of the formula:

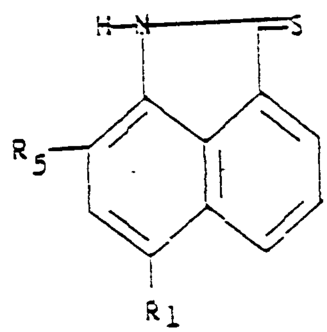

wherein $R_1$ and $R_5$ are as defined above
with a substituted (1H-imidazol-1-yl)alkanamine of the formula

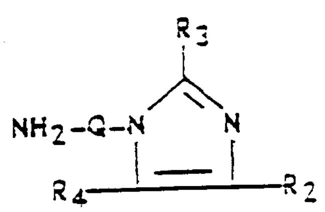

wherein $R_2$, $R_3$, $R_4$ and Q are as defined above
and mercuric oxide or mercuric acetate in a solvent such as ethanol at reflux temperature for 2-24 hours.

**25.** A process for producing a compound according to Claim 1 and the pharmacologically acceptable salts which comprises reacting a substituted 2-methylthiobenz[cd]indole salt of the structure:

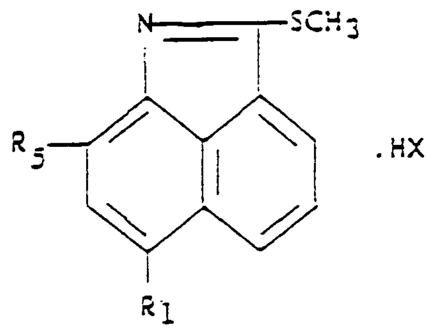

wherein $R_1$ and $R_5$ are as defined in Claim 1, where X is halogen, methylsulfate or sulfate with a substituted (1H-imidazol-1-yl)alkanamine of the formula:

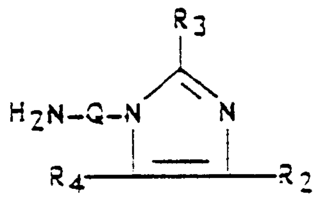

wherein $R_2$, $R_3$, $R_4$ and Q are as defined in Claim 1, in a solvent such as ethanol at reflux temperature for 6-24 hours.

**Claims for the following Contracting States : AT, ES, GR**

1. A process for producing a compound having the formula:

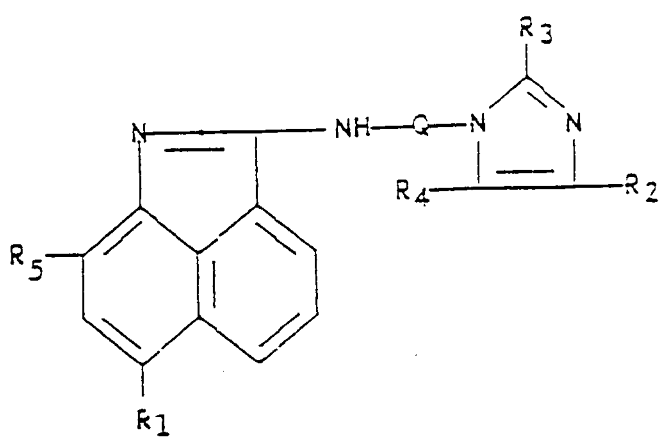

wherein $R_1$ is hydrogen, bromo, chloro or dimethylsulfonamide; $R_2$ is hydrogen, or alkyl($C_1$-$C_3$); $R_3$ is hydrogen, alkyl ($C_1$-$C_3$), or phenyl; $R_4$ is hydrogen or when taken together with $R_2$ is -CH=CH-CH=CH-; $R_5$ is hydrogen or chloro; and Q is $-(CH_2)_n-$, where n is an integer from 2 through 12,

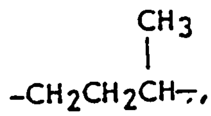, 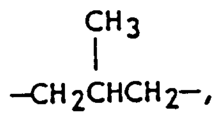, 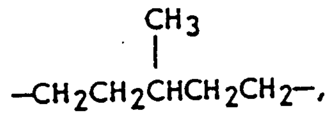,

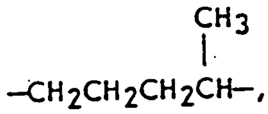, 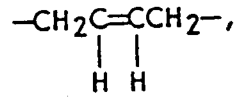, 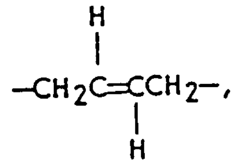,

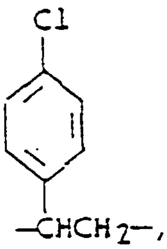, 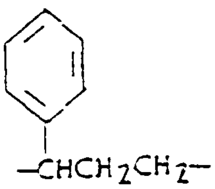, 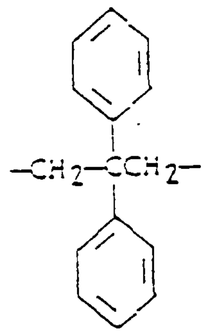,

$-CH_2CH_2-O-CH_2CH_2-$, 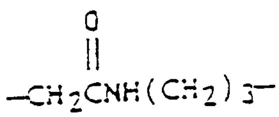 or 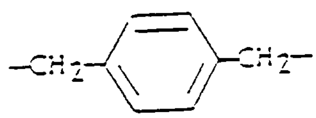

or a pharmacologically acceptable salt thereof which comprises reacting a substituted benz[cd]indol-2-thiol of the formula:

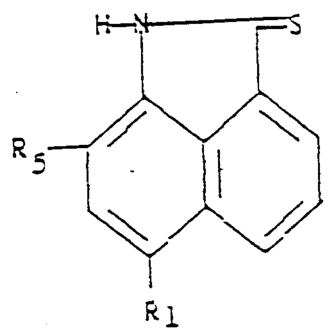

wherein $R_1$ and $R_5$ are as defined above
with a substituted (1H-imidazol-1-yl)alkanamine of the formula

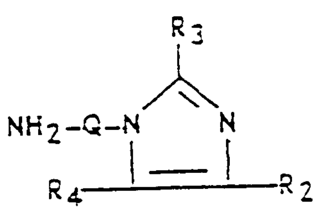

wherein $R_2$, $R_3$, $R_4$ and Q are as defined above and mercuric oxide or mercuric acetate in a solvent such as ethanol at reflux temperature for 2-24 hours.

2. A process for producing a compound according to Claim 1 and the pharmacologically acceptable salts which comprises reacting a substituted 2-methylthiobenz[cd]indole salt of the structure:

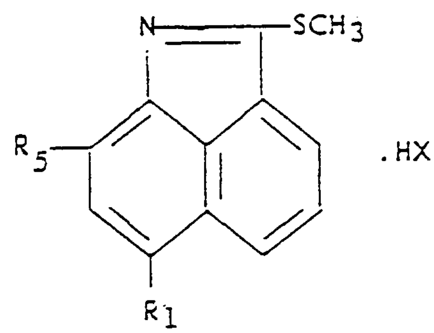

wherein $R_1$ and $R_5$ are as defined in Claim 1, where X is halogen, methylsulfate or sulfate with a substituted (1H-imidazol-1-yl)alkanamine of the formula:

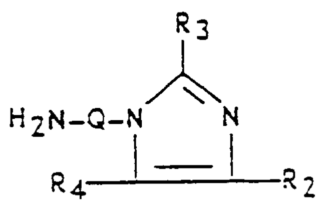

wherein $R_2$, $R_3$, $R_4$ and Q are as defined in Claim 1, in a solvent such as ethanol at reflux temperature for 6-24 hours.

3. The method according to claim 1. which produces N-[3-(3-pyridinyl)propyl]benz[cd]indol-2-amine fumarate.

4. The method according to claim 1. which produces N-[3-(3-pyridinyloxy)propyl]benz[cd]indol-2-amine fumarate.

5. The method according to claim 1. which produces 6-bromo-N-[4-(3-pyridinyl)butyl]benz[cd]indol-2-amine sesqui-fumarate.

6. The method according to claim 1. which produces N-[4-(3-pyridinyl)]benz[cd]indol-2-amine, hydriodide salt.

7. Method according to claim 1. which produces N-[4-(3-pyridinyl)]benz[cd]indol-2-amine, sesquifumarate salt.

8. Method according to claim 1 which produces N-[2-(3-Pyridinyl)ethyl]benz[cd]indol-2-amine sesquifumarate.

9. Method according to claim 1. which produces N-[4-(1H-imidazol-1-yl)butyl]benz[cd]indol-2-amine.

10. Method according to claim 1 which produces N-[3-(1H-imidazol-1-yl)propyl]benz[cd]indol-2-amine.

11. Method according to claim 1. which produces 6-bromo-N-[3-(1H-imidazol-1-yl)butyl]benz[cd]indol-2-amine.

12. Method according to claim 1 which produces 6-bromo-N-[5-(1H-imidazol-1-yl)pentyl]benz[cd]indol-2-amine.

13. Method according to claim 1. which produces 6,8-dichloro-N-[5-(1H-imidazol-1-yl)pentyl]benz[cd]indol-2-amine.

14. Method according to claim 1. which produces 6-chloro-N-[3-(1H-imidazol-1-yl)butyl]benz[cd]indol-2-amine.

15. Method according to claim 1 which produces N-[3-(1H-imidazol-1-yl)-1-phenylpropyl]benz[cd]indol-2-amine.

16. Method according to claim 1. which produces N-[3-(1H-imidazol-1-yl)-2-methylpropyl]benz[cd]indol-2-amine.

17. Method according to claim 1 which produces (E)-N-[4-(1H-imidazol-1-yl)-2-butenyl]benz[cd]indol-2-amine.

18. Method according to claim 1. which produces 2-{[3-(1H-imidazol-1-yl)butyl]amino}-N,N-dimethylbenz[cd]indol-6-sulfonamide.

19. Method according to claim 1 which produces 2-{[3-(1H-imidazol-1-yl)propyl]amino}-N,N-dimethylbenz[cd]indol-6-sulfonamide.

20. Method according to claim 1. which produces 2-benz[cd]indole-2-ylamino)-N-[3-(1H-imidazol-1-yl)propyl]acetamide.

21. Method according to claim 1. which produces (Z)-N-[4-(1H-imidazol-1-yl)-2-butenyl]benz[cd]indol-2-amine.

22. Method according to claim 1 which produces N-[5-(1H-imidazol-1-yl)-3-methylpentyl]benz[cd]indol-2-amine.

23. Method according to claim 1. which produces 6,8-dichloro-N-[3-(1H-imidazol-1-yl)propyl]benz[cd]indol-2-amine.

## Patentansprüche

### Patentansprüche für folgende Vertragsstaaten : BE, CH, DE, FR, GB, IT, LI, NL, SE

1. Verbindung der Formel:

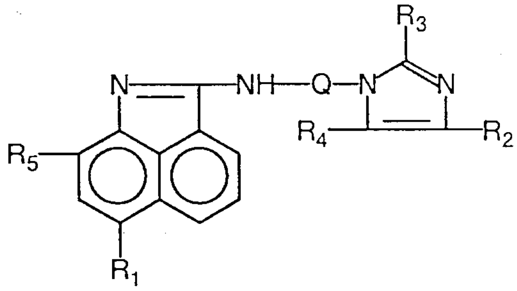

worin $R_1$ Wasserstoff, Brom, Chlor oder Dimethylsulfonamid ist, $R_2$ Wasserstoff oder $C_1$-$C_3$-Alkyl ist, $R_3$ Wasserstoff, $C_1$-$C_3$-Alkyl oder Phenyl ist, $R_4$ Wasserstoff oder, zusammengenommen mit $R_2$, -CH=CH-

CH = CH- ist, $R_5$ Wasserstoff oder Chlor und Q $-(CH_2)_n-$ , worin n eine ganze Zahl von 2 bis 12 ist,

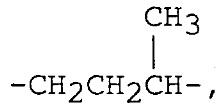, 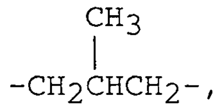, 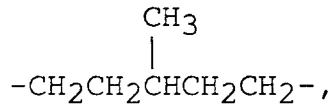,

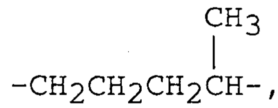, 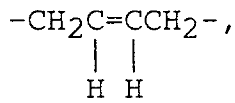, 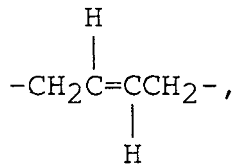,

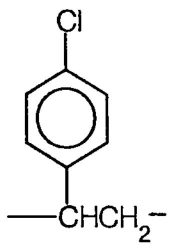, 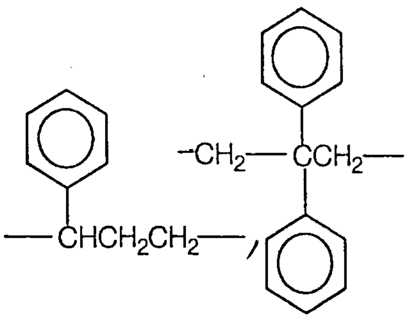,

$-CH_2CH_2-O-CH_2CH_2-$, 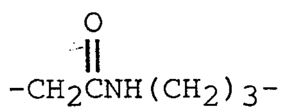 oder

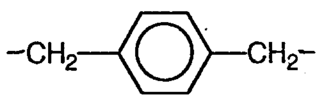

ist und deren pharmakologisch akzeptable Salze.

**2.** Die Verbindung
N-[3-(3-Pyridinyl)propyl]benz[cd]indol-2-amin-fumarat.

**3.** Die Verbindung
N-[3-(3-Pyridinyloxy)propyl]benz[cd]indol-2-amin-fumarat.

**4.** Die Verbindung
6-Brom-N-[4-(3-pyridinyl)butyl]benz[cd]indol-2-amin-sesquifumarat.

**5.** Die Verbindung
N-[4-(3-Pyridinyl)]benz[cd]indol-2-amin-hydroiodidsalz.

**6.** Die Verbindung
N-[4-(3-Pyridinyl)]benz[cd]indol-2-amin-sesquifumaratsalz.

**7.** Die Verbindung
N-[2-(3-Pyridinyl)ethyl]benz[cd]indol-2-amin-sesquifumarat.

**8.** Die Verbindung nach Anspruch 1,
N-[4-(1H-Imidazol-1-yl)butyl]benz[cd]indol-2-amin.

**9.** Die Verbindung nach Anspruch 1,
N-[4-(1H-Imidazol-1-yl)propyl]benz[cd]indol-2-amin.

**10.** Die Verbindung nach Anspruch 1,
6-Brom-N-[3-(1H-imidazol-1-yl)butyl]benz[cd]indol-2-amin.

**11.** Die Verbindung nach Anspruch 1,
6-Brom-N-[5-(1H-imidazol-1-yl)pentyl]benz[cd]indol-2-amin.

**12.** Die Verbindung nach Anspruch 1,
6,8-Dichlor-N-[5-(1H-imidazol-1-yl)butyl]benz[cd]indol-2-amin.

**13.** Die Verbindung nach Anspruch 1,
6-Chlor-N-[3-(1H-imidazol-1-yl)butyl]benz[cd]indol-2-amin.

**14.** Die Verbindung nach Anspruch 1,
N-[3-(1H-Imidazol-1-yl)-1-phenylpropyl]benz[cd]indol-2-amin.

**15.** Die Verbindung nach Anspruch 1,
N-[3-(1H-Imidazol-1-yl)-2-methylpropyl]benz[cd]indol-2-amin.

**16.** Die Verbindung nach Anspruch 1,
(E)-N-[4-(1H-Imidazol-1-yl)-2-butenyl]benz[cd]indol-2-amin.

**17.** Die Verbindung nach Anspruch 1,
2-{[3-(1H-Imidazol-1-yl)butyl]amino}-N,N-dimethylbenz[cd]indol-6-sulfonamid.

**18.** Die Verbindung nach Anspruch 1,
2-{[3-(1H-Imidazol-1-yl)butyl]amino}-N,N-dimethylbenz[cd]indol-6-sulfonamid.

**19.** Die Verbindung nach Anspruch 1,
2-Benz[cd]indol-2-ylamino)-N-[3-(1H-imidazol-1-yl)propyl]acetamid.

**20.** Die Verbindung nach Anspruch 1,
(Z)-N-[4-(1H-Imidazol-1-yl)-2-butenyl]benz[cd]indol-2-amin.

**21.** Die Verbindung nach Anspruch 1,
N-[5-(1H-Imidazol-1-yl)-3-methylpentyl]benz[cd]indol-2-amin.

**22.** Die Verbindung nach Anspruch 1,
6,8-Dichlor-N-[3-(1H-imidazol-1-yl)propyl]benz[cd]-indol-2-amin.

**23.** Thromboxan-Synthetase-Enzym hemmende Zusammensetzung in Form von Dosierungseinheiten, umfassend 10 mg bis 700 mg einer Verbindung nach Anspruch 1 in Verbindung mit einem pharmakologisch akzeptablen Träger.

**24.** Verfahren zum Herstellen einer Verbindung nach Anspruch 1 der Formel:

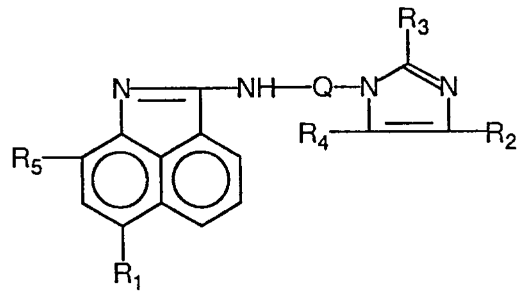

worin $R_1$, $R_2$, $R_3$, $R_4$, $R_5$ und Q die in Anspruch 1 genannte Bedeutung haben und von deren pharmakologisch akzeptablen Salzen, umfassend
Umsetzen eines substituierten Benz[cd]indol-2-thiols der Formel:

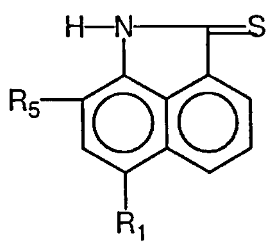

worin $R_1$ und $R_5$ die oben genannte Bedeutung haben,
mit einem substituierten (1H-Imidazol-1-yl)alkanamin der Formel:

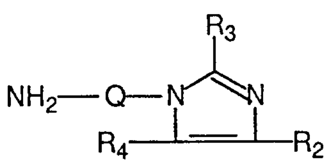

worin $R_2$, $R_3$, $R_4$ und Q die oben genannte Bedeutung haben, und Quecksilber(II)oxid oder Quecksilber(II)acetat in einem Lösungsmittel, wie Ethanol, bei Rückflußtemperatur für 2 bis 24 Stunden.

**25.** Verfahren zum Herstellen einer Verbindung nach Anspruch 1 und von deren pharmakologisch akzeptablen Salzen, umfassend
Umsetzen eines substituierten 2-Methylthiobenz[cd]indolsalzes der Formel:

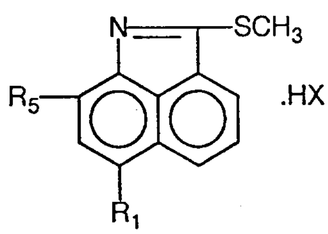

worin $R_1$ und $R_5$ die in Anspruch 1 genannte Bedeutung haben, X Chlor, Methylsulfat oder Sulfat ist,
mit einem substituierten (1H-Imidazol-1-yl)alkanamin der Formel:

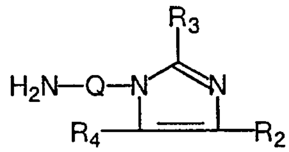

worin $R_2$, $R_3$, $R_4$ und Q die in Anspruch 1 angegebene Bedeutung haben, in einem Lösungsmittel, wie Ethanol, bei Rückflußtemperatur für 6 bis 24 Stunden.

**Patentansprüche für folgende Vertragsstaaten : AT, ES, GR**

**1.** Verfahren zum Herstellen einer Verbindung der Formel:

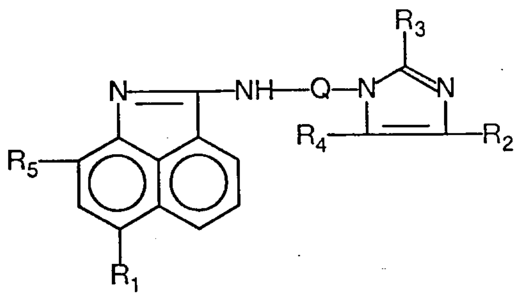

worin $R_1$ Wasserstoff, Brom, Chlor oder Dimethylsulfonamid ist, $R_2$ Wasserstoff oder $C_1$-$C_3$-Alkyl ist, $R_3$ Wasserstoff, $C_1$-$C_3$-Alkyl oder Phenyl ist, $R_4$ Wasserstoff oder, zusammengenommen mit $R_2$, -CH = CH-CH = CH- ist, $R_5$ Wasserstoff oder Chlor und Q $-(CH_2)_n-$ , worin n eine ganze Zahl von 2 bis 12 ist,

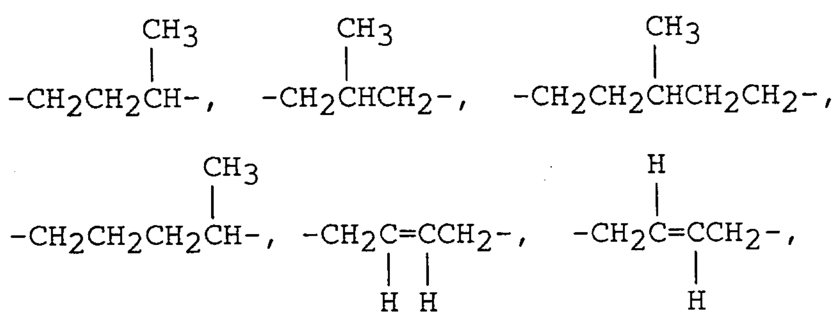

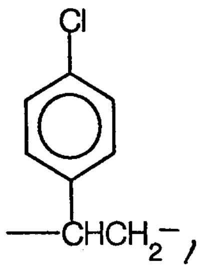, 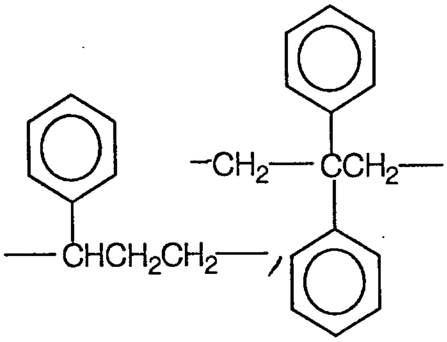

$-CH_2CH_2-O-CH_2CH_2-$, $-CH_2\overset{O}{\overset{\|}{C}}NH(CH_2)_3-$ oder

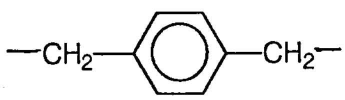

ist und deren pharmakologisch akzeptable Salze, umfassend Umsetzen eines substituierten Benz[cd]-indol-2-thiols der Formel:

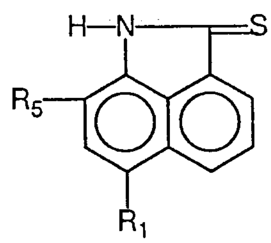

worin $R_1$ und $R_5$ die oben genannte Bedeutung haben,
mit einem substituierten (1H-Imidazol-1-yl)alkanamin der Formel:

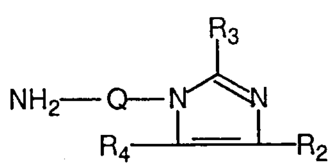

worin $R_2$, $R_3$, $R_4$ und Q die oben genannte Bedeutung haben, und Quecksilber(II)oxid oder Quecksilber-(II)acetat in einem Lösungsmittel, wie Ethanol, bei Rückflußtemperatur für 2 bis 24 Stunden.

**2.** Verfahren zum Herstellen einer Verbindung nach Anspruch 1 und deren pharmakologisch akzeptable Salze, umfassend Umsetzen eines substituierten 2-Methylthiobenz[cd]indolsalzes der Formel:

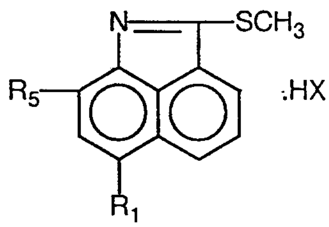

worin $R_1$ und $R_5$ die in Anspruch 1 genannte Bedeutung haben, X Chlor, Methylsulfat oder Sulfat ist, mit einem substituierten (1H-Imidazol-1-yl)alkanamin der Formel:

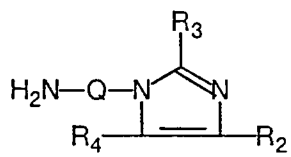

worin $R_2$, $R_3$, $R_4$ und Q die in Anspruch 1 angegebene Bedeutung haben, in einem Lösungsmittel, wie Ethanol, bei Rückflußtemperatur für 6 bis 24 Stunden.

**3.** Verfahren nach Anspruch 1, das erzeugt N-[3-(3-Pyridinyl)propyl]benz[cd]indol-2-amin-fumarat.

**4.** Verfahren nach Anspruch 1, das erzeugt N-[3-(3-Pyridinyloxy)propyl]benz[cd]indol-2-amin-fumarat.

**5.** Verfahren nach Anspruch 1, das erzeugt 6-Brom-N-[4-(3-pyridinyl)butyl]benz[cd]indol-2-amin-sesqui-fumarat.

**6.** Verfahren nach Anspruch 1, das erzeugt N-[4-(3-Pyridinyl)]benz[cd]indol-2-amin-hydroiodidsalz.

**7.** Verfahren nach Anspruch 1, das erzeugt N-[4-(3-Pyridinyl)]benz[cd]indol-2-amin-sesquifumaratsalz.

**8.** Verfahren nach Anspruch 1, das erzeugt N-[2-(3-Pyridinyl)ethyl]benz[cd]indol-2-amin-sesqui-fumarat.

**9.** Verfahren nach Anspruch 1, das erzeugt N-[4-(1H-Imidazol-1-yl)butyl]benz[cd]indol-2-amin.

**10.** Verfahren nach Anspruch 1, das erzeugt N-[3-(1H-Imidazol-1-yl)butyl]benz[cd]indol-2-amin.

**11.** Verfahren nach` Anspruch 1, das erzeugt 6-Brom-N-[3-(1H-Imidazol-1-yl)butyl]benz[cd]indol-2-amin.

**12.** Verfahren nach Anspruch 1, das erzeugt 6-Brom-N-[5-(1H-Imidazol-1-yl)pentyl]benz[cd]indol-2-amin.

**13.** Verfahren nach Anspruch 1, das erzeugt 6,8-Dichlor-N-[5-(1H-imidazol-1-yl)pentyl]benz[cd]indol-2-amin.

**14.** Verfahren nach Anspruch 1, das erzeugt 6-Chlor-N-[3-(1H-imidazol-1-yl)butyl]benz[cd]indol-2-amin.

**15.** Verfahren nach Anspruch 1, das erzeugt N-[3-(1H-Imidazol-1-yl)-1-phenylpropyl]benz[cd]indol-2-amin.

**16.** Verfahren nach Anspruch 1, das erzeugt N-[3-(1H-Imidazol-1-yl)-2-methylpropyl]benz[cd]indol-2-amin.

**17.** Verfahren nach Anspruch 1, das erzeugt (E)-N-[4-(1H-Imidazol-1-yl)-2-butenyl]benz[cd]indol-2-amin.

**18.** Verfahren nach Anspruch 1, das erzeugt 2-{[3-(1H-Imidazol-1-yl)butyl]amino}-N,N-dimethylbenz[cd]indol-6-sulfonamid.

**19.** Verfahren nach Anspruch 1, das erzeugt 2-{[3-(1H-Imidazol-1-yl)propyl]amino}-N,N-dimethylbenz[cd]indol-6-sulfonamid.

**20.** Verfahren nach Anspruch 1, das erzeugt 2-Benz[cd]indol-2-ylamino)-N-[3-(1H-imidazol-1-yl)propyl]acetamid.

**21.** Verfahren nach Anspruch 1, das erzeugt (Z)-N-[4-(1H-Imidazol-1-yl)-2-butenyl]benz[cd]indol-2-amin.

**22.** Verfahren nach Anspruch 1, das erzeugt N-[5-(1H-Imidazol-1-yl)-3-methylpentyl]benz[cd]indol-2-amin.

**23.** Verfahren nach Anspruch 1, das erzeugt 6,8-Dichloro-N-[3-(1H-imidazol-1-yl)propyl]benz[cd]indol-2-amin.

**Revendications**

**Revendications pour les Etats contractants suivants : BE, CH, DE, FR, GB, IT, LI, NL, SE**

**1.** Un composé ayant la formule:

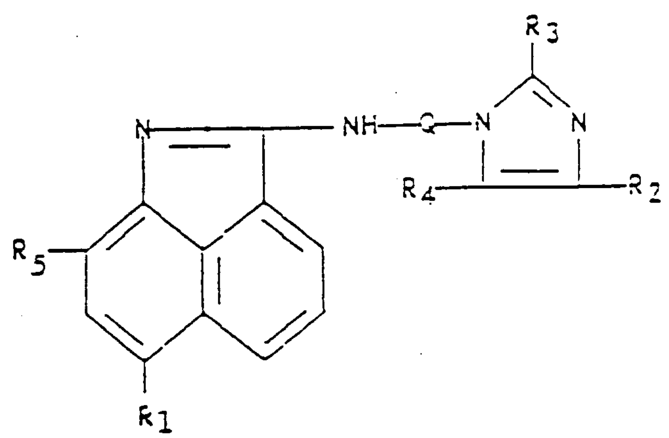

dans laquelle $R_1$ est un atome d'hydrogène, de brome, de chlore ou un groupe diméthylsulfonamide ; $R_2$ est un atome d'hydrogène ou un groupe alkyle ($C_1$-$C_3$) ; $R_3$ est un atome d'hydrogène, un groupe alkyle ($C_1$-$C_3$) ou phényle ; $R_4$ est un atome d'hydrogène ou lorsqu'il est pris ensemble avec $R_2$ est un groupe -CH=CH-CH=CH- ; $R_5$ est un atome d'hydrogène ou de chlore ; et Q est $-(CH_2)_n-$, où n est un nombre entier de 2 à 12,

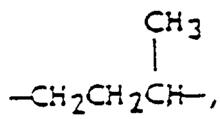, 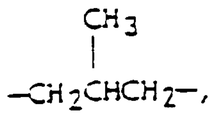, 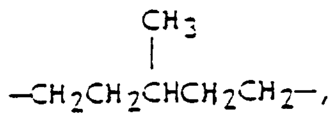,

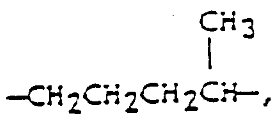, 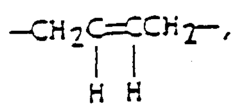, 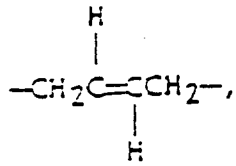,

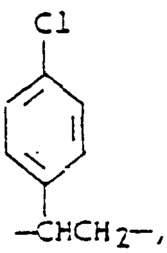, 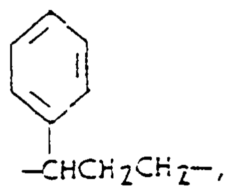, 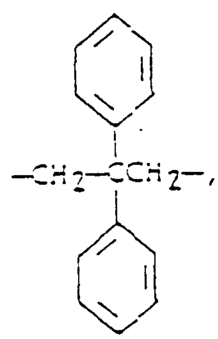,

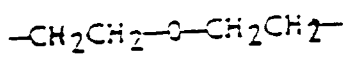, 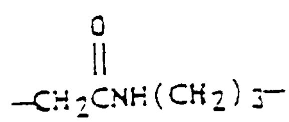 ou

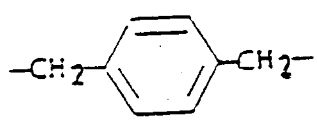

et les sels pharmacologiquement acceptables de ces composés.

2. Le composé N-[3-(3-pyridinyl)propyl]benz[cd]indol-2-amine, fumarate.

3. Le composé N-[3-pyridinyloxy)propyl]benz[cd]indol-2-amine, fumarate.

4. Le composé 6-bromo-N-[4-(3-pyridinyl)butyl]benz[cd]indol-2-amine, sesqui-fumarate.

5. Le composé N-[4-(3-pyridinyl)]benz[cd]indol-2-amine, sel iodhydrate.

6. Le composé N-[4-(3-pyridinyl)]benz[cd]indol-2-amine, sel sesqui-fumarate.

7. Le composé N-[2-(3-pyridinyl)éthyl]benz[cd]indol-2-amine, sesqui-fumarate.

8. Le composé selon la revendication 1, la N-[4-(1H-imidazole-1-yl)butyl]butyl]benz[cd]indole-2-amine.

9. Le composé selon la revendication 1, la N-[3-(1H-imidazole-1-yl)propyl]benz[cd]indole-2-amine.

10. Le composé selon la revendication 1, la 6-bromo-N-[3-(1H-imidazole-1-yl)butyl]benz[cd]indole-2-amine.

**11.** Le composé selon la revendication 1, la 6-bromo-N-[5-(1H-imidazole-1-yl)pentyl]benz[cd]indole-2-amine.

**12.** Le composé selon la revendication 1, la 6,8-dichloro-N-[5-(1H-imidazole-1-yl)pentyl]benz[cd]-indole-2-amine.

**13.** Le composé selon la revendication 1, la 6-chloro-N-[3-(1H-imidazole-1-yl)butyl]benz[cd]indole-2-amine.

**14.** Le composé selon la revendication 1, la N-[3-(1H-imidazole-1-yl)-1-phénylpropyl]benz[cd]indole-2-amine.

**15.** Le composé selon la revendication 1, la N-[3-(1H-imidazole-1-yl)-2-méthylpropyl]benz[cd]indole-2-amine.

**16.** Le composé selon la revendication 1, la (E)-N-[4-(1H-imidazole-1-yl)-2-butényl]benz[cd]indole-2-amine.

**17.** Le composé selon la revendication 1, la 2-{[3-(1H-imidazole-1-yl)butyl]amino}-N,N-diméthylbenz[cd]-indole-6-sulfonamide.

**18.** Le composé selon la revendication 1, le 2-{[3-(1H-imidazole-1-yl)propyl]amino}-N,N-diméthylbenz[cd]-indole-6-sulfonamide.

**19.** Le composé selon la revendication 1, le 2-benz[cd]indole-2-ylamino)-N-[3-(1H-imidazole-1-yl)propyl]-acétamide.

**20.** Le composé selon la revendication 1, le (Z)-N-[4-(1H-imidazole-1-yl)-2-buténynl]benz[cd]indole-2-amine.

**21.** Le composé selon la revendication 1, la N-[5-(1H-imidazole-1-yl)-3-méthylpentyl]benz[cd]indole-2-amine.

**22.** Le composé selon la revendication 1, la 6,8-dichloro-N-[3-(1H-imidazole-1-yl)propyl]benz[cd]indole-2-amine.

**23.** Une composition de matière inhibitrice de l'enzyme thromboxane synthétase sous forme de dosage unitaire comprenant de 10 mg à 700 mg d'un composé selon la revendication 1, en association avec un support pharmaceutique acceptable.

**24.** Un procédé de production d'un composé selon la revendication 1, de formule :

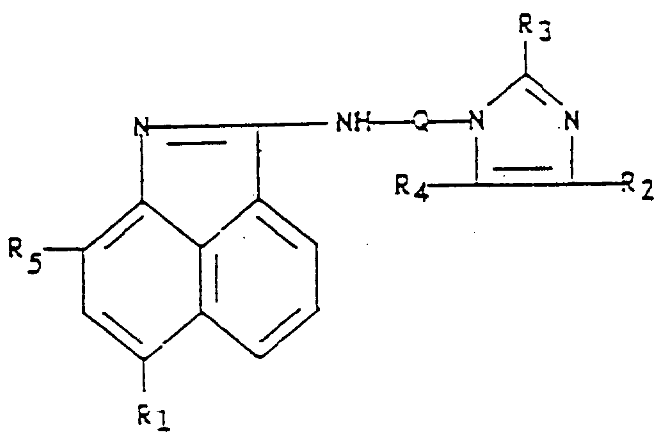

dans laquelle $R_1$, $R_2$, $R_3$, $R_4$, $R_5$ et Q sont comme défini dans la revendication 1, et ses sels pharmacologiquement acceptables qui comprend la réaction d'un benz[cd]indole-2-thiol substitué de formule :

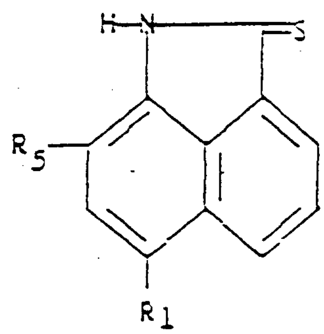

dans laquelle $R_1$ et $R_5$ sont comme défini précédemment avec une (1H-imidazole-1-yl)alcanamine substituée de formule :

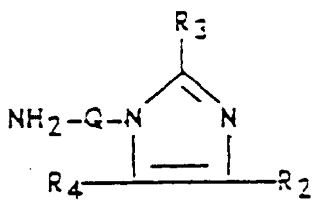

dans laquelle $R_2$, $R_3$, $R_4$ et Q sont comme défini ci-dessus et avec l'oxyde mercurique ou l'acétate mercurique dans un solvant tel que l'éthanol à la température de reflux pendant 2-24h.

**25.** Un procédé pour la production d'un composé selon la revendication 1 et les sels pharmacologiquement acceptables dudit composé qui comprend la réaction d'un sel de 2-méthylthiobenz[cd]indole substitué de structure :

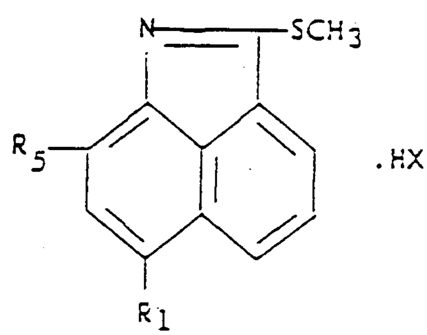

dans laquelle $R_1$ et $R_5$ sont comme défini dans la revendication 1, et X est un atome d'halogène, un groupe méthylsulfate ou sulfate avec une (1H-imidazole-1-yl)alcanamine substituée de formule :

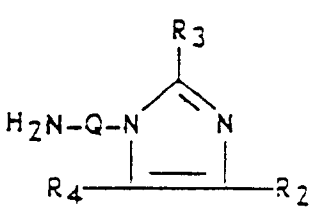

dans laquelle $R_2$, $R_3$, $R_4$ et Q sont comme défini dans la revendication 1, dans un solvant tel que l'éthanol à la température de reflux pendant 6-24 h.

**Revendications pour les Etats contractants suivants : AT, ES, GR**

**1.** Un procédé de production d'un composé ayant la formule :

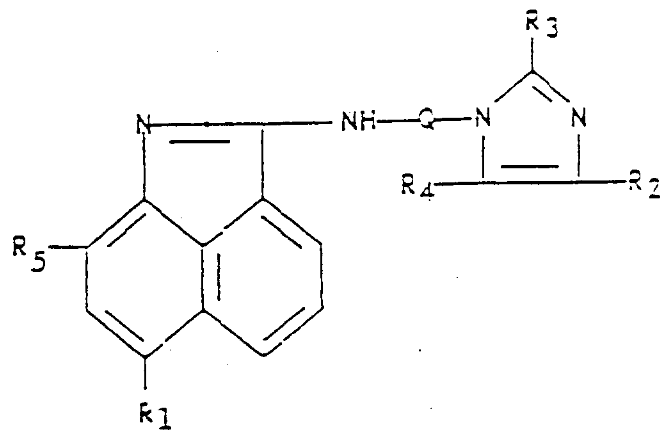

dans laquelle $R_1$ est un atome d'hydrogène, de brome, de chlore ou un groupe diméthylsulfonamide ; $R_2$ est un atome d'hydrogène ou un groupe alkyle ($C_1$-$C_3$); $R_3$ est un atome d'hydrogène, un groupe alkyle ($C_1$-$C_3$) ou phényle ; $R_4$ est un atome d'hydrogène ou lorsqu'il est pris ensemble avec $R_2$ est un groupe -CH = CH = CH = CH- ; $R_5$ est un atome d'hydrogène ou de chlore ; et Q est $-(CH_2)_n-$, où n est un nombre entier de 2 à 12,

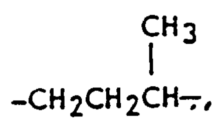
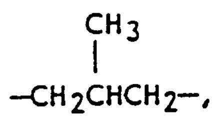
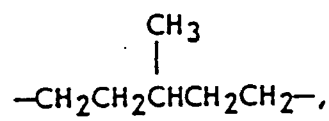
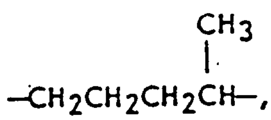
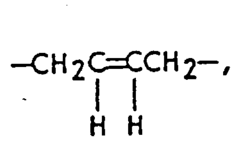
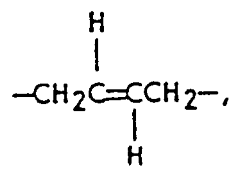
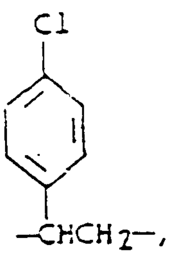
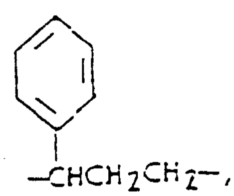
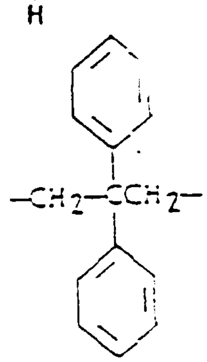

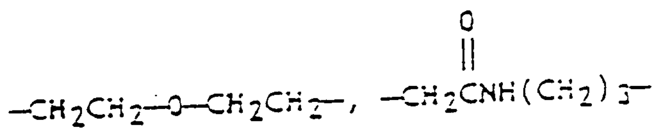 ou

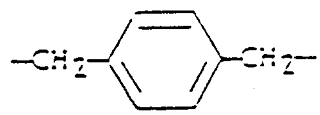

et les sels pharmacologiquement acceptables de ces composés qui comprend la réaction d'un benz[cd]indole-2-thiol substitué de formule :

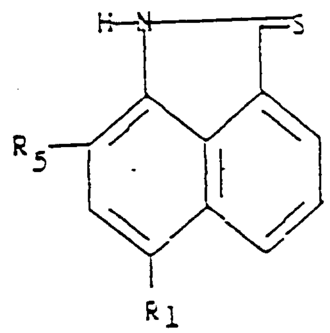

dans laquelle $R_1$ et $R_5$ sont comme défini ci-dessus avec une (1H-imidazole-1-yl)alcanamine substituée de formule :

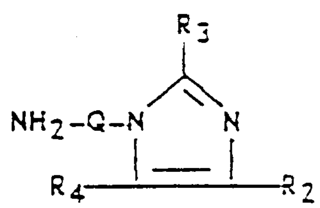

dans laquelle $R_2$, $R_3$, $R_4$ et Q sont comme défini ci-dessus et avec l'oxyde mercurique ou l'acétate mercurique dans un solvant tel que l'éthanol à la température de reflux pendant 2-24 h.

2. Un procédé de production d'un composé selon la revendication 1, et les sels pharmacologiquement acceptables dudit composé qui comprend la réaction d'un sel de 2-méthylthiobenz[cd]indole substitué de structure :

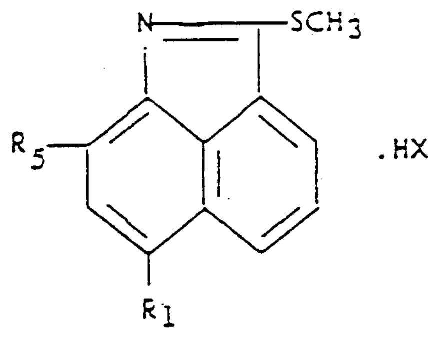

dans laquelle $R_1$ et $R_5$ sont comme défini dans la revendication 1, X est un atome d'halogène, un

groupe méthylsulfate ou sulfate avec une (1H-imidazole-1-yl)-alcanamine substituée de formule :

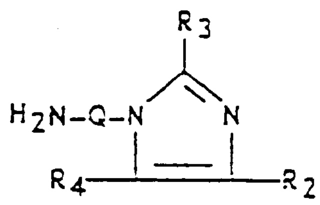

dans laquelle $R_2$, $R_3$, $R_4$ et Q sont comme défini dans la revendication 1, dans un solvant tel que l'éthanol à la température de reflux pendant 6-24 h.

**3.** Le procédé selon la revendication 1, qui produit le N-[3-(3-pyridinyl)propyl]benz[cd]indole-2-amine, fumarate.

**4.** Le procédé selon la revendication 1, qui produit le N-[3-pyridinyloxy)propyl]benz[cd[indole-2-amine, fumarate.

**5.** Le procédé selon la revendication 1, qui produit le 6-bromo-N-[4-(3-pyridinyl)butyl]benz[cd]indole-2-amine, sesqui-fumarate.

**6.** Le procédé selon la revendication 1, qui produit le N-[4-(3-pyridinyl)]benz[cd]indole-2-amine, sel iodhydrate.

**7.** Le procédé selon la revendication 1, qui produit le N-[4-(3-piridinyl)]benz[cd]indole-2-amine, sel sesqui-fumarate.

**8.** Le procédé selon la revendication 1, qui produit le N-[2-(3-pyridinyl)éthyl]benz[cd]indole-2-amine, sesqui-fumarate.

**9.** Le procédé selon la revendication 1, qui produit la N-[4-(1H-imidazole-1-yl)butyl]butyl]benz[cd]indole-2-amine.

**10.** Le procédé selon la revendication 1, qui produit la N-[3-(1H-imidazole-1-yl)propyl]benz[cd]indole-2-amine.

**11.** Le composé selon la revendication 1, qui produit la 6-bromo-N-[3-(1H-imidazole-1-yl)butyl]benz[cd]-indole-2-amine.

**12.** Le procédé selon la revendication 1, qui produit la 6-bromo-N-[5-(1H-imidazole-1-yl)pentyl]benz[cd]-indole-2-amine.

**13.** Le procédé selon la revendication 1, qui produit la 6,8-dichloro-N-[5-(1H-imidazole-1-yl)pentyl]benz[cd]-indole-2-amine.

**14.** Le procédé selon la revendication 1, qui produit la 6-chloro-N-[3-(1H-imidazole-1-yl)butyl]benz[cd]-indole-2-amine.

**15.** Le procédé selon la revendication 1, qui produit la N-[3-(1H-imidazole-1-yl)-1-phénylpropyl]benz[cd]-indole-2-amine.

**16.** Le procédé selon la revendication 1, qui produit la N-[3-(1H-imidazole-1-yl)-2-méthylpropyl]benz[cd]-indole-2-amine.

**17.** Le procédé selon la revendication 1, qui produit la (E)-N-[4-(1H-imidazole-1-yl)-2-buténylbenz[cd]-indole-2-amine.

18. Le procédé selon la revendication 1, qui produit la 2-{[3-(1H-imidazole-1-yl)butyl]amino}-N,N-diméthylbenz[cd]indole-6-sulfonamide.

19. Le procédé selon la revendication 1, qui produit le 2-{[3-(1H-imidazole-1-yl)propyl]amino}-N,N-diméthylbenz[cd]indole-6-sulfonamide.

20. Le procédé selon la revendication 1, qui produit le 2-benz[cd]indole-2-ylamino)-N-[3-(1H-imidazole-1-yl)propyl]acétamide.

21. Le procédé selon la revendication 1, qui produit le (Z)-N-[4-(1H-imidazole-1-yl)-2-buténylJbenz[cd]-indole-2-amine.

22. Le procédé selon la revendication 1, qui produit la N-[5-(1H-imidazole-1-yl)-3-méthylpentyl]benz[cd]-indole-2-amine.

23. Le procédé selon la revendication 1, qui produit la 6,8-dichloro-N-[3-(1H-imidazole-1-yl)propyl]benz[cd]-indole-2-amine.